# EUROPÄISCHE PATENTSCHRIFT

(11) **EP 2 582 691 B1**
(45) Veröffentlichungstag und Bekanntmachung des Hinweises auf die Patenterteilung: **10.05.2017**
(21) Anmeldenummer: 11724250.3
(22) Anmeldetag: 10.06.2011
(51) Int. Cl.: C07D 401/06, C07D 403/06, C07D 413/06, C07D 417/06, A01N 43/56, A01N 43/713, A01N 43/76, A01N 43/78, A01P 7/00

(54) **ANTHRANILSÄUREDERIVATE**
ANTHRANILIC ACID DERIVATIVES
DÉRIVÉS D'ACIDE ANTHRANILIQUE

(30) Priorität: 15.06.2010 US 354895 P; 15.06.2010 EP 10166062
(43) Veröffentlichungstag der Anmeldung: 24.04.2013
(73) Patentinhaber: Bayer Intellectual Property GmbH, 40789 Monheim (DE)
(72) Erfinder: FISCHER, Rüdiger, 50259 Pulheim (DE); GRONDAL, Christoph, 50937 Köln (DE); HEIL, Markus, 42799 Leichlingen (DE); WROBLOWSKY, Heinz-Juergen, 40764 Langenfeld (DE); GESING, Ernst, Rudolf, 40699 Erkrath (DE); VOERSTE, Arnd, 50674 Köln (DE); GÖRGENS, Ulrich, 40882 Ratingen (DE)
(86) Internationale Anmeldenummer: PCT/EP2011/059696
(87) Internationale Veröffentlichungsnummer: WO 2011/157653

(56) Entgegenhaltungen:
- EP-A1- 1 911 751
- WO-A1-2007/068356
- WO-A1-2007/144100
- WO-A1-2011/128329
- WO-A2-01/70671

## Beschreibung

Die vorliegende Erfindung betrifft neue Anthranilsäurederivate, deren Anwendung als Insektizide und Akarizide zur Bekämpfung tierischer Schädlinge, auch in Kombination mit weiteren Mitteln zur Wirkungssteigerung, und mehrere Verfahren zu ihrer Herstellung.

Anthranilsäurederivate mit insektiziden Eigenschaften sind in der Literatur bereits beschrieben, z.B. in WO 01/70671, WO 03/015519, WO 03/016284, WO 03/015518, WO 03/024222, WO 03/016282, WO 03/016283, WO 03/062226, WO 03/027099, WO 04/027042, WO 04/033468, WO 2004/046129, WO 2004/067528, WO 2005/118552, WO 2005/077934, WO 2005/085234, WO 2006/023783, WO 2006/000336, WO 2006/040113, WO 2006/111341, WO 2007/006670, WO 2007/024833, WO2007/020877, WO 2007/144100, WO2007/043677, WO2008/126889, WO2008/126890 und WO2008/126933.

Die gemäß den oben genannten Schriften bereits bekannten Wirkstoffe weisen aber in ihrer Anwendung teils Nachteile auf, sei es, dass sie nur eine geringe Anwendungsbreite aufweisen, sei es, dass sie keine zufriedenstellende insektizide oder akarizide Wirkung aufweisen.

Es wurden nun neue Anthranilsäurederivate gefunden, welche gegenüber den bereits bekannten Verbindungen Vorteile aufweisen, z.B. seien bessere biologische oder ökologische Eigenschaften, breitere Anwendungsmethoden, eine bessere insektizide, akarizide Wirkung, sowie eine gute Verträglichkeit gegenüber Nutzpflanzen beispielhaft genannt. Die Anthranilsäurederivate können in Kombination mit weiteren Mitteln zur Verbesserung der Wirksamkeit insbesondere gegen schwierig zu bekämpfende Insekten eingesetzt werden.

Gegenstand der vorliegenden Erfindung sind daher neue Anthranilsäurederivate der Formel (I) 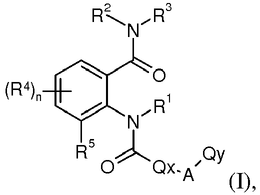 in welcher
- R¹: für Wasserstoff steht,
- R²: für Wasserstoff steht.
- R³: für Wasserstoff, C₁-C₄-Alkyl (Methyl, Ethyl, n-Propyl, iso-Propyl, n-Butyl, iso-Butyl, sec-Butyl oder tert-Butyl), cyclo-Propyl, cyclo-Butyl, Cyano-C₁-C₃-Alkyl (Cyanomethyl, 1-Cyanoethyl, 2-Cyanoethyl, 1-Cyano-n-Propyl, 2-Cyano-n-Propyl, 3-Cyano-n-Propyl, 1-Cyano-iso-Propyl, 2-Cyano-iso-Propyl), Phenyl, Pyridyl, oder
- R⁴: für Wasserstoff, Methyl, Trifluormethyl, Cyano, Fluor, Chlor, Brom, Iod oder Trifluormethoxy steht. Ausserdem stehen zwei benachbarte Reste R⁴ für -(CH₂)₄-, oder -(CH=CH-)₂-.
- n: für 0 bis 3 steht,
- R⁵: für Methyl, Fluor, Chlor, Brom oder Iod steht,
- Qx: für Thiazol, Oxazol, Pyrrol, Imidazol, Triazol, Pyrimidin, Phenyl oder für Pyrazol steht, wobei der Rest R⁷ über Kohlenstoff an das Pyrazol gebunden ist,
welches durch die Gruppe R⁷ 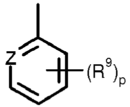
- A: für -CH₂-, -CH(CH₃), C(CH₃)₂, -CH₂CH₂-, -CH(CN)-, -CH₂O- oder -C(=O)-CH₂-steht,
- R⁷: für den Rest 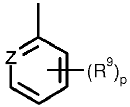
steht,
- R⁹: unabhängig voneinander für Fluor, Chlor oder Brom steht,
- p: für 1 steht,
- Z: für N, CCl oder CH steht,
- Q_{Y}: für einen gegebenenfalls einfach oder mehrfach substituierten 5- oder 6-gliedrigen heteroaromatischen Ring der Reihe Q-36 bis Q-40 ,Q43, Q-58 bis Q-59, Q62, Q63, einen aromatisches 9-gliedriges annelliertes heterobicyclisches Ringsystem Q-54 bis Q-56 sowie für ein 5-gliedrigen heterocyclischen Ring Q-60 bis Q-61 steht, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy,
oder wobei die Substituenten unabhängig voneinander ausgewählt sein können aus Phenyl oder einem 5-oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können. 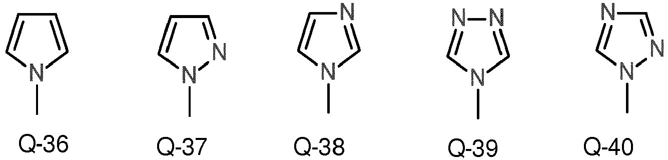 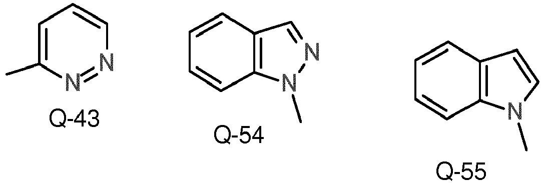 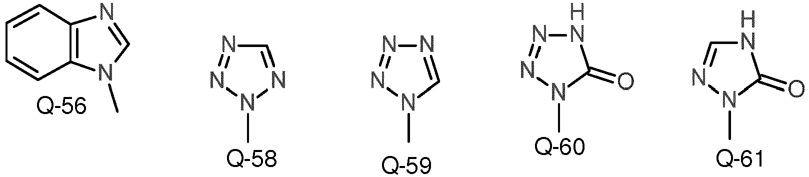 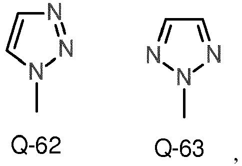 die Verbindungen der allgemeinen Formel (I) außerdem N-Oxide und Salze umfassen.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der Formel (I) umfassen gegebenenfalls Diastereomere oder Enantiomere.

Die erfindungsgemäßen Verbindungen sind durch die Formel (I) allgemein definiert. Bevorzugt, besonders bevorzugt und ganz besonders sind Verbindungen der Formel (I), in welcher
R³ insbesondere bevorzugt für Wasserstoff, Methyl, iso-Propyl, cyclo-Propyl, tert-Butyl oder Cyanomethyl steht.
R⁴ steht insbesondere bevorzugt für Chlor, Fluor oder Brom,
R⁴ steht weiterhin insbesondere bevorzugt für Iod oder Cyano.
zwei benachbarte Reste R⁴ stehen insbesondere bevorzugt für -(CH=CH-)₂
R⁵ insbesondere bevorzugt für Methyl oder Chlor steht,
A insbesonders bevorzugt für CH₂, CH(CH₃), -CH₂O- oder -C(=O)-CH₂- steht,
R⁹ insbesondere bevorzugt für Chlor steht,
Q_{Y} insbesonders bevorzugt für einen gegebenenfalls einfach oder mehrfach gleich oder verschieden substituierten heteroaromatischen Ring der Reihe Q-37, Q-38, Q-39, Q-40, Q43, Q-58, Q-59, Q62 und Q63, sowie für ein 5-gliedrigen heterocyclischen Ring Q-60 steht, wobei die Substituenten unabhängig voneinander ausgewählt sein können aus Methyl, Ethyl, cyclo-Propyl, tert-Butyl, Chlor, Fluor, Iod, Brom, Cyano, Nitro, Difluormethyl, Trifluormethyl, Pentafluorethyl., n-Heptafluorpropyl und iso-Heptafluorpropyl
oder wobei die Substituenten unabhängig voneinander ausgewählt sein können aus Phenyl oder einem 5- oder 6-gliedrigen heteroaromatischen Ring, wobei die Substituenten unabhängig voneinander ausgewählt sein können aus Methyl, Ethyl, cyclo-Propyl, tert-Butyl, Chlor, Fluor, Iod, Brom, Cyano, Nitro, Difluormethyl, Trifluormethyl, Pentafluorethyl., n-Heptafluorpropyl und iso-Heptafluorpropyl,

Die oben aufgeführten Ringe oder Ringsysteme können gegebenenfalls unabhängig voneinander zusätzlich durch Oxo, Thioxo, (=O)=NH, (=O)=N-CN, (=O)₂ substituiert sein. Beispielhaft seien genannt Tetrahydrothiophendioxid, Imidazolidon.

Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen -N(O)-, -S(O)- (auch kurz SO) und -S(O)₂- (auch kurz SO₂) im heterocyclischen Ring. Im Fall von -N(O)- und -S(O)-Gruppen sind jeweils beide Enantiomere umfasst.

Andere Substituenten als die Oxogruppe können an einem heterocyclischen Ring auch an einem Heteroatom gebunden sein, beispielsweise an einem Stickstoffatom, wenn dabei ein Wasserstoffatom am Stickstoffatom des Grundkörpers ersetzt wird. Im Falle des Stickstoffatoms und auch anderer Heteroatome wie z. B. des Schwefelatoms, kommt auch eine weitere Substitution unter Bildung von quartären Ammoniumverbindungen oder Sulfoniumverbindungen in Frage.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Insbesondere können die Verbindungen der Formeln (I) in Form verschiedener Regioisomere vorliegen. Beispielsweise in Form von Mischungen aus Verbindungen, wobei die Bindung des Rings oder des Ringsystems Qy an den Rest (A) jeweils über unterschiedliche Kohlenstoff- oder Stickstoffatome erfolgt.

Weiterhin beispielsweise in Form von Mischungen aus Verbindungen mit der Definition Q62 und Q63 oder in Form von Mischungen aus Verbindungen mit der Definition Q58 und Q59. Erfindungsgemäß umfasst sind daher auch Mischungen aus Verbindungen der Formeln (I), wobei Q_{Y} die Bedeutungen Q62 und Q63, sowie Q58 und Q59 hat und die Verbindungen in verschiedenen Mischungsverhältnissen vorliegen können. Bevorzugt sind dabei Mischungsverhältnisse aus Verbindungen der Formel (I), worin der Rest Q_{Y} für Q62 oder für Q58 steht zu Verbindungen der Formel (I) worin der Rest Q_{Y} für Q63 oder für Q59 steht, von 60:40 bis 99:1, besonders bevorzugt von 70:30 bis 98:2, ganz besonders bevorzugt von 80:20 bis 97:3. Insbesonders bevorzugt sind die folgenden Mischungsverhältnisse einer Verbindung der Formel (I), wobei Q_{Y} die Bedeutung Q62 oder Q58 hat zur Verbindung der Formel (I), wobei Q_{Y} die Bedeutung Q63 oder Q59 hat: 80:20; 81:19; 82:18; 83:17; 84:16; 85:15, 86:14; 87:13; 88:12; 89:11; 90:10, 91:9; 92:8; 93:7; 94:6; 95;5; 96:4; 97:3.

### Herstellverfahren

Die Verbindungen der allgemeinen Formel (I) können erhalten werden, in dem man
(A) Aniline der Formel (II) 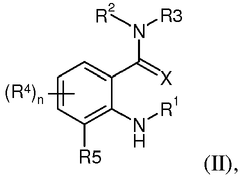 in welcher R¹, R², R³, R⁴, R⁵ und n die oben angegebenen Bedeutungen haben und X für O steht, beispielsweise mit Carbonsäurechloriden der Formel (III) 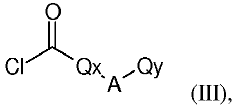 wobei
   Qx, A und Qy die oben angegebenen Bedeutungen haben,
   in Gegenwart eines Säurebindemittels umsetzt; oder
(B) Aniline der Formel (II) 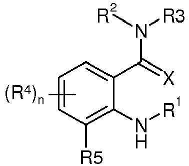 in welcher R¹, R², R³, R⁴, R⁵ und n die oben angegebenen Bedeutungen haben und X für O steht, beispielsweise mit einer Carbonsäure der Formel (IV) 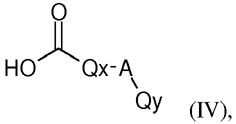 wobei
   Qx, A und Qy die oben angegebenen Bedeutungen haben,
   in Gegenwart eines Kondensationsmittels umsetzt; oder
(C) zur Synthese von Anthranilamiden der Formel (I), in welcher R¹ für Wasserstoff steht, beispielsweise Benzoxazinone der Formel (V) 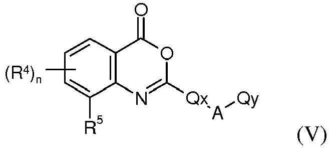 in welcher R⁴, R⁵, Qx, A, Qy und n die oben angegebenen Bedeutungen haben, mit einem Amin der Formel (VI) 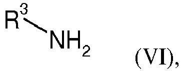 in welcher R³ die oben angegebenen Bedeutungen hat,
   in Gegenwart eines Verdünnungsmittels umsetzt zu erfindungsgemäßen Verbindungen der Formel (I).

Insbesondere können Verbindungen der allgemeinen Formel (I), in welchen Qx für 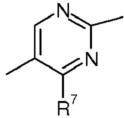 steht, hergestellt werden, indem man
(B-1) Aniline der Formel (II) 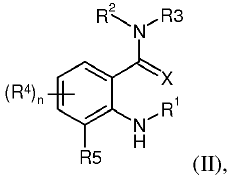 in welcher R¹, R², R³, R⁴, R⁵ X und n die oben angegebenen Bedeutungen haben, beispielsweise mit einer Carbonsäure der Formel (IV-1) 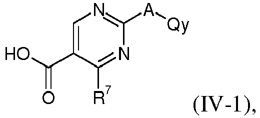 in welcher R⁷, A und Qy die oben angegebenen Bedeutungen haben, in Gegenwart eines Kondensationsmittels umsetzt.

Carbonsäuren der Formel (IV-1) können nach dem folgenden Reaktionsschema, in welchem R⁷, A, Qy die oben angegebenen Bedeutungen haben und R für C₁-C₆ Alkyl und Hal für Halogen steht aus Verbindungen der Formel (VIII) hergestellt werden. Verbindungen der Formel (VIII) sind bekannt (z.B. J. Med. Chem. 49, 2006, 4721-4736). Die Umsetzung von (VIII) zu (IX) kann nach bekannten Methoden durchgeführt werden (z.B. WO2005/113506). Die weitere Umsetzung über Verbindungen der Formel X zu Verbindungen der Formel (IV-1) kann nach bekannten Methoden durchgeführt werden (z.B. WO2007/144100).

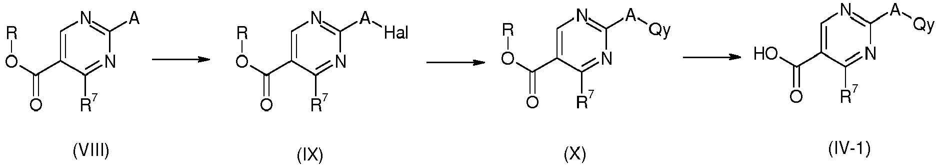

Insbesondere können Verbindungen der allgemeinen Formel (I), in welchen Qx für 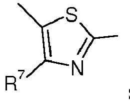 steht, hergestellt werden, indem man
(B-2) Aniline der Formel (II) 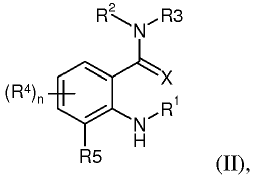 in welcher R¹, R², R³, R⁴, R⁵, X und n die oben angegebenen Bedeutungen haben, beispielsweise mit einer Carbonsäure der Formel (IV-2)

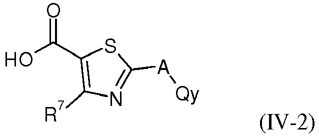

in welcher R⁷, A und Qy die oben angegebenen Bedeutungen haben,
in Gegenwart eines Kondensationsmittels umsetzt.

Carbonsäuren der Formel (IV-2) können nach dem folgenden Reaktionsschema, in welchem R⁷, A und Qy die oben angegebenen Bedeutungen haben und R für C₁-C₆ Alkyl und Hal für Halogen steht aus Verbindungen der Formel (XI) hergestellt werden. Verbindungen der Formel (XI) sind bekannt (z.B. J. Med. Chem. 49, 2006, 4721-4736). Die Umsetzung von (XI) zu (XII) kann nach bekannten Methoden durchgeführt werden (z.B. WO2005/113506). Die weitere Umsetzung über XIII zu Carbonsäuren der Formel (IV-2) kann nach bekannten Methoden durchgeführt werden (z.B. WO2007/144100).

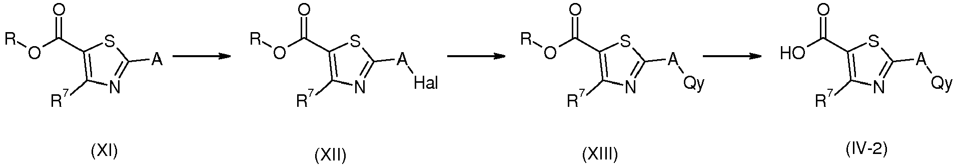

Insbesondere können Verbindungen der allgemeinen Formel (I), in welchen Qx für 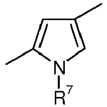 steht,
hergestellt werden, indem man
(B-3) Aniline der Formel (II) 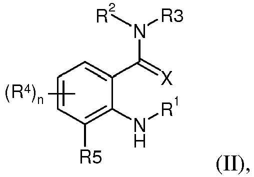 in welcher R¹, R², R³, R⁴, R⁵, X und n die oben angegebenen Bedeutungen haben, beispielsweise mit einer Carbonsäure der Formel (IV-3) 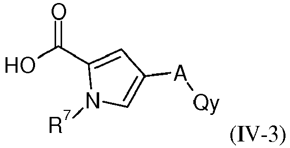 in welcher R⁷, A und Qy die oben angegebenen Bedeutungen haben, in Gegenwart eines Kondensationsmittels umsetzt.

Carbonsäuren der Formel (IV-3) sind neu. Sie können nach dem folgenden Reaktionsschema, in welchem R⁷ und Q_{y} die oben angegebenen Bedeutungen haben und R für C₁-C₆ Alkyl steht aus Verbindungen der Formel XIV hergestellt werden. Verbindungen der Formel XIV sind bekannt (z.B. WO 2003016283). Die Umsetzung von Verbindungen der Formel XIV zu Verbindungen der Formel XV kann nach bekannten Methoden durchgeführt werden wie z.B. mit (1-Ethoxyvinyl)-tributylstannan unter Palladiumkatalyse (z.B. J. Med. Chem. 41, 1998, 3736). Die weitere Umsetzung zu Verbindungen dr Formel XVI erfolgt mit einem geeigneten Halogenierungsmittel (z.B Bioorganic & Medicinal Chemistry Letters, 19(4), 1199-1205; 2009). Die Kupplung mit Qy zu Verbindungen der Formel XVII und die Verseifung zu Carbonsäuren der Formel IV-3 erfolgen nach bekannten Methoden (z.B. Bioorganic & Medicinal Chemistry, 17(6), 2410-2422; 2009 und WO2006004903).

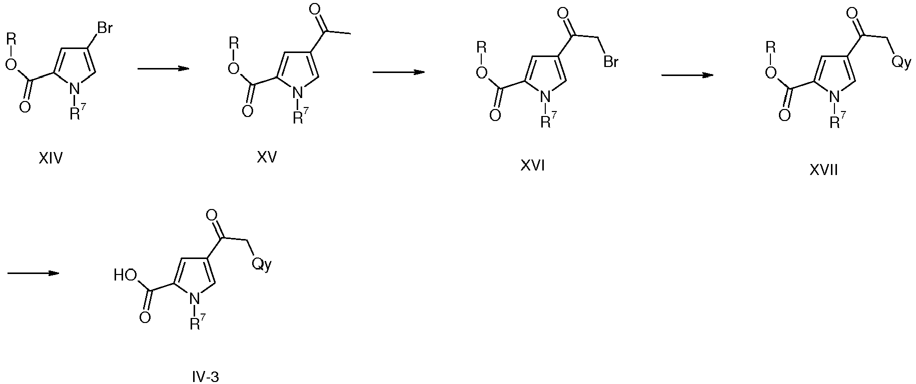

Insbesondere können Verbindungen der allgemeinen Formel (I), in welchen Qx für 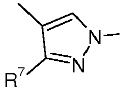 steht, hergestellt werden, indem man
(B-4) Aniline der Formel (II) 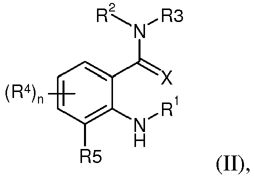 in welcher R¹, R², R³, R⁴, R⁵, X und n die oben angegebenen Bedeutungen haben, beispielsweise mit einer Carbonsäure der Formel (IV-4) 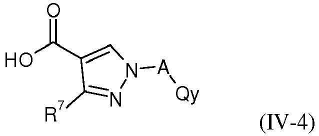 in welcher R⁷, A und Qy die oben angegebenen Bedeutungen haben, in Gegenwart eines Kondensationsmittels umsetzt.

Carbonsäuren der Formel (IV-4) können nach dem folgenden Reaktionsschema, in welchem R⁷, A und Qy die oben angegebenen Bedeutungen haben und R für C₁-C₆ Alkyl und Hal für Halogen steht, aus Verbindungen der Formel (XVIII) mit (XIX) hergestellt werden. Verbindungen der Formel (XVIII) sind bekannt (z.B. WO 2007 043677) und käuflich erhältlich. Verbindungen der Formel (XIX) sind käuflich erhältlich. Die Alkylierung von (XVIII) mit (XIX) kann nach bekannten Methoden durchgeführt werden (z.B: Bioorg. Med. Chem. 2008, 16, 10165-10171). Die weitere Umsetzung von Verbindungen der Formel (XX) zu Carbonsäuren der Formel (IV-4) kann nach bekannten Methoden durchgeführt werden (z.B. WO2007/144100).

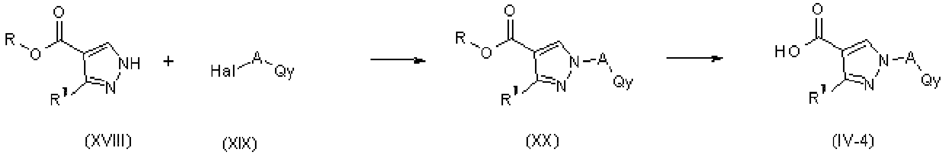

Insbesondere können Verbindungen der allgemeinen Formel (I), in welchen Qx für 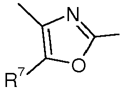 steht, hergestellt werden, indem man
(B-5) Aniline der Formel (II) 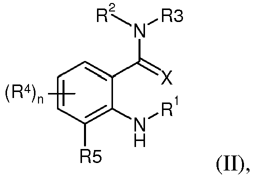 in welcher R¹, R², R³, R⁴, R⁵, X und n die oben angegebenen Bedeutungen haben, beispielsweise mit einer Carbonsäure der Formel (IV-5) 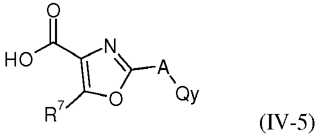 in welcher R⁷, A und Qy die oben angegebenen Bedeutungen haben, in Gegenwart eines Kondensationsmittels umsetzt.

Carbonsäuren der Formel (IV-5) können nach dem folgenden Reaktionsschema, in welchem R⁷, A und Qy die oben angegebenen Bedeutungen haben und R für C₁-C₆ Alkyl und Hal für Halogen steht, aus Verbindungen der Formel (XXI) hergestellt werden. Verbindungen der Formel (XXI) sind bekannt und können nach bekannten Methoden hergestellt werden (z.B. J. Med. Chem. 2006, 49, 4721-4736). Die Umsetzung von (XXI) zu (XXII) kann nach bekannten Methoden durchgeführt werden (z.B. WO2005/113506). Die weitere Umsetzung über (XXIII) zu Carbonsäuren der Formel (IV-5) kann nach bekannten Methoden durchgeführt werden (z.B. WO2007/144100).

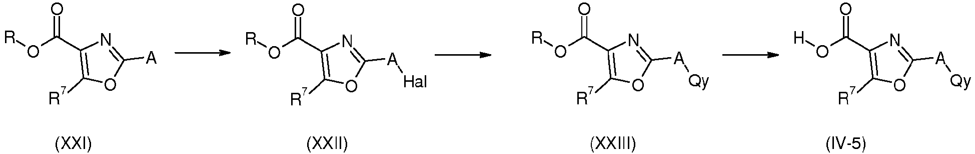

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die Wirksamkeit der Verbindungen der Formel (I) lässt sich durch die Zugabe von Ammonium- und Phosphoniumsalzen steigern. Die Ammonium- und Phosphoniumsalze werden durch Formel (XI) definiert 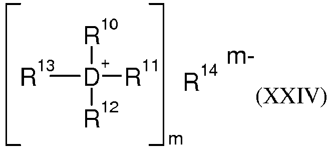 in welcher
D für Stickstoff oder Phosphor steht,
D bevorzugt für Stickstoff steht,
R¹⁰, R¹¹, R¹², and R¹³ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
R¹⁰, R¹¹, R¹², and R¹³ bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
R¹⁰, R¹¹, R¹², and R¹³ besonders bevorzugt unabhängig voneinander für Wasserstoff, Methyl, Ethyl, n-Propyl, i-Propyl, n-Butyl, i-Butyl, s-Butyl oder t-Butyl stehen,
R¹⁰, R¹¹, R¹², and R¹³ ganz besonders bevorzugt für Wasserstoff stehen,
m für 1, 2, 3 oder 4 steht,
m bevorzugt für 1 oder 2 steht,
R¹⁴ für ein anorganisches oder organisches Anion steht,
R¹⁴ bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat, Citrat oder Oxalat steht,
R¹⁴ besonders bevorzugt für Laktat, Sulfat, Monohydrogenphosphat, Dihydrogenphosphat, Nitrat, Thiosulfat, Thiocyanat, Citrat, Oxalat oder Formiat steht.
R¹⁴ ganz besonders bevorzugt für Sulfat steht.

Die Ammonium- und Phosphoniumsalze der Formel (XXIV) können in einem breiten Konzentrationsbereich zur Steigerung der Wirkung von Pflanzenschutzmitteln enthaltend Verbindungen der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern ein Penetrationsförderer zugegeben. Selbst in diesen Fällen ist eine Wirkungssteigerung zu beobachten. Gegenstand der vorliegenden Erfindung ist also ebenfalls die Verwendung eines Penetrationsförders, sowie die Verwendung einer Kombination von Penetrationsförderer und Ammonium- und/oder Phosphoniumsalzen zur Wirkungssteigerung von Pflanzenschutzmitteln, die akarizid/insektizid wirksame Verbindungen der Formel (I) als Wirkstoff enthalten. Gegenstand der Erfindung ist schließlich weiterhin die Verwendung dieser Mittel zur Bekämpfung von Schadinsekten.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel

R-O-(-AO)ᵥ-R' (XXV)

in welcher
R für geradkettiges oder verzweigtes Alkyl mit 4 bis 20 Kohlenstoffatomen steht,
R' für Wasserstoff, Methyl, Ethyl, n-Propyl, i-Propyl, n-Butyl, i-Butyl, t-Butyl, n-Pentyl oder n-Hexyl steht,
AO für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
v für Zahlen von 2 bis 30 steht.

Eine bevorzugte Gruppe von Penetrationsförderern sind Alkanolalkoxylate der Formel

R-O-(-EO-)ₙ-R' (XXV-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (XXV-b)

in welcher
- R: die oben angegebene Bedeutung hat,
- R': die oben angegebene Bedeutung hat,
- EO: für -CH₂-CH₂-O- steht,

- PO: für 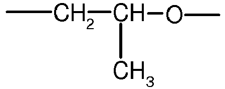
steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (XXV-c)

in welcher
- R: die oben angegebene Bedeutung hat,
- R': die oben angegebene Bedeutung hat,
- EO: für -CH₂-CH₂-O- steht,

- PO: für 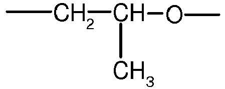
steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (XXV-d)

in welcher
- R und R': die oben angegebenen Bedeutungen haben,
- EO: für CH₂-CH₂-O- steht,

- BO: für 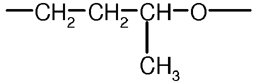
steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (XXV-e)

in welcher
- R und R': die oben angegebenen Bedeutungen haben,
- BO: für 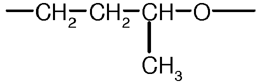
steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (XXV-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (XXV-c) sei 2-Ethyl-hexyl-alkoxylat der Formel 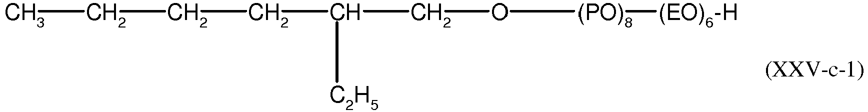 in welcher
- EO: für -CH₂-CH₂-O- steht,
- PO: für 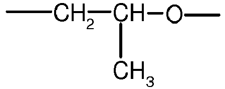
steht und die Zahlen 8 und 6 Durchschnittswerte darstellen, genannt.

Als Beispiel für ein Alkanol-Alkoxylat der Formel (XII-d) sei die Formel

CH₃-(CH₂)₁₀-O-(-EO-)₆-(-BO-)₂-CH₃ (XXV-d-1)

in welcher
- EO: für CH₂-CH₂-O- steht,

- BO: für 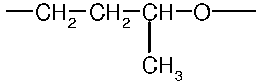
steht und
die Zahlen 10, 6 und 2 Durchschnittswerte darstellen, genannt.

Besonders bevorzugte Alkanol-Alkoxylate der Formel (XXV-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (XXV-f-1)

CH₃-(CH₂)ₜ-CH₂-O-(-CH₂-CH₂-O-)ᵤ-R' (XXV-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Löslichkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäß hervorgehobene Kombinationen von Wirkstoff, Salz und Penetrationsförderer sind in folgender Tabelle aufgeführt. "Gemäß Test" bedeutet dabei, dass jede Verbindung geeignet ist, die in dem Test für die Kutikelpenetration (Baur et al., 1997, Pesticide Science 51, 131-152) als Penetrationsförderer wirkt:

| # | Wirkstoff | Salz | Penetrationsförderer |
|---|---|---|---|
| 1 | I | Ammoniumsulfat | Gemäß Test |
| 2 | I | Ammoniumlaktat | Gemäß Test |
| 3 | I | Ammoniumnitrat | Gemäß Test |
| 4 | I | Ammoniumthiosulfat | Gemäß Test |
| 5 | I | Ammoniumthiocyanat | Gemäß Test |
| 6 | I | Ammoniumcitrat | Gemäß Test |
| 7 | I | Ammoniumoxalat | Gemäß Test |
| 8 | I | Ammoniumformiat | Gemäß Test |
| 9 | I | Ammoniumhydrogenphosphat | Gemäß Test |
| 10 | I | Ammoniumdihydrogenphosphat | Gemäß Test |
| 11 | I | Ammoniumcarbonat | Gemäß Test |
| 12 | I | Ammoniumbenzoat | Gemäß Test |
| 13 | I | Ammoniumsulfit | Gemäß Test |
| 14 | I | Ammoniumbenzoat | Gemäß Test |
| 15 | I | Ammoniumhydrogenoxalat | Gemäß Test |
| 16 | I | Ammoniumhydrogencitrat | Gemäß Test |
| 17 | I | Ammoniumacetat | Gemäß Test |
| 18 | I | Tetramethylammoniumsulfat | Gemäß Test |
| 19 | I | Tetramethylammoniumlaktat | Gemäß Test |
| 20 | I | Tetramethylammoniumnitrat | Gemäß Test |
| 21 | I | Tetramethylammoniumthiosulfat | Gemäß Test |
| 22 | I | Tetramethylammoniumthiocyanat | Gemäß Test |
| 23 | I | Tetramethylammoniumcitrat | Gemäß Test |
| 24 | I | Tetramethylammoniumoxalat | Gemäß Test |
| 25 | I | Tetramethylammoniumformiat | Gemäß Test |
| 26 | I | Tetramethylammoniumhydrogenphosphat | Gemäß Test |
| 27 | I | Tetramethylammoniumdihydrogenphosphat | Gemäß Test |
| 28 | I | Tetraethylammoniumsulfat | Gemäß Test |
| 29 | I | Tetraethylammoniumlaktat | Gemäß Test |
| 30 | I | Tetraethylammoniumnitrat | Gemäß Test |
| 31 | I | Tetraethylammoniumthiosulfat | Gemäß Test |
| 32 | I | Tetraethylammoniumthiocyanat | Gemäß Test |
| 33 | I | Tetraethylammoniumcitrat | Gemäß Test |
| 34 | I | Tetraethylammoniumoxalat | Gemäß Test |
| 35 | I | Tetraethylammoniumformiat | Gemäß Test |
| 36 | I | Tetraethylammoniumhydrogenphosphat | Gemäß Test |
| 37 | I | Tetraethylammoniumdihydrogenphosphat | Gemäß Test |

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Herstellverfahren und Zwischenprodukte

Aniline der Formel (II) sind bekannt (z.B. WO2007/043677, WO2008/126858, WO2008/126933) oder können nach bekannten Methoden hergestellt werden.

### Synthese von Carbonsäuren der Formel (IV-1)

### Beispiel Nr. (IX)

### Synthese von Ethyl-2-(brommethyl)-4-(2-chlorphenyl)pyrimidin-5-carboxylat

820 mg (2,96 mmol) Ethyl-4-(2-chlorphenyl)-2-methylpyrimidin-5-carboxylat und 633 mg (3,55 mmol) N-Brom-succinimid wurden in 18 mL Tetrachlorkohlenstoff gelöst und unter Rühren zum sieden erhitzt. In der Siedehitze wurden portionsweise 49 mg (0,29 mmol) 2,2'-Azobis-2-methylpropannitril über 5 Stunden zugegeben und für eine weitere Stunde refluxiert. Nach Abkühlen auf Raumtemperatur wurde die Reaktionslösung filtriert und die Mutterlauge im Vakuum vom Lösungsmittel befreit. Durch chromatographische Reinigung wurde das gewünschte Produkt isoliert.

(logP: 3,32; MH⁺: 357; ¹H-NMR (400 MHz, DMSO, δ, ppm): 1,00 (t, 3H), 4,12 (q, 2H), 4,80 (s, 2H), 7,50 (m, 4 H), 9,30 (s, 1H).

### Beispiel Nr. (X)

### Synthese von Ethyl-4-(2-chlorphenyl)-2-{[5-(trifluormethyl)-2H-tetrazol-2-yl]methyl}pyrimidin-5-carboxylat

345 mg (0,97 mmol) Ethyl-2-(brommethyl)-4-(2-chlorphenyl)pyrimidin-5-carboxylat wurden zu einer Lösung aus 200 mg (1,45 mmol) Trifluormethyltetrazol in 10 mL Acetonitril gegeben. Anschließend wurden 174 mg (1,26 mmol) Kaliumcarbonat zur Reaktionslösung gegeben und für 6 Stunden bei 60 °C gerührt. Nach Abkühlen auf Raumtemperatur wurde die Reaktionslösung filtriert und die Mutterlauge im Vakuum vom Lösungsmittel befreit. Der Rückstand wurde mit 10 mL Wasser versetzt und gegen Essigester (3 x 20 mL) extrahiert. Die vereinigten organischen Phasen wurden über Natriumsulfat getrocknet. Das Lösungsmittel wurde im Vakuum entfernt und der Rückstand chromatographisch aufgereinigt. (logP: 3,56; MH⁺: 413; ¹H-NMR (400 MHz, DMSO, δ, ppm): 0,99 (t, 3H), 4,13 (q, 2H), 6,62 (s, 2H), 7,50 (m, 4 H), 9,28 (s, 1H).

### Beispiel Nr. (IV-1)

### Synthese von 4-(2-Chlorphenyl)-2-{[5-(trifluormethyl)-2H-tetrazol-2-yl]methyl}pyrimidin-5-carbonsäure

1,74 g (4,21 mmol) Ethyl-4-(2-chlorphenyl)-2-{[5-(trifluormethyl)-2H-tetrazol-2-yl]methyl}pyrimidin-5-carboxylat wurden in 12 mL Ethanol gelöst und bei 0 °C tropfenweise mit 45%iger Natronlauge (5,05 mmol) versetzt. Die Reaktion wurde eine Stunde nachgerührt und auf RT erwärmt. Anschließend wurde das Ethanol unter vermindertem Druck entfernt und der Rückstand wurde mit Eiswasser (10 mL) versetzt. Die wässrige Phase wurde gegen Essigester extrahiert. Die organische Phase wurde verworfen. Anschließend wurde die wässrige Phase mit Salzsäure auf pH 3 eingestellt und nochmals gegen Essigester (3 x 20 mL) extrahiert. Die vereinigten organischen Phasen wurden über Natriumsulfat getrocknet. (logP: 2,50; MH⁺: 385; ¹H-NMR (400 MHz, DMSO, δ, ppm): 6,59 (s, 2H), 7,48 (m, 4 H), 9,27 (s, 1H).

### Synthese von Carbonsäuren der Formel IV-2

### Beispiel Nr. XII

### Synthese von Methyl-2-(brommethyl)-4-(2-chlorphenyl)-1,3-thiazol-5-carboxylat

5,00 g (18,6 mmol) Methyl-4-(2-chlorphenyl)-2-methyl-1,3-thiazol-5-carboxylat und 3,38 g (19,0 mmol) N-Brom-succinimid wurden in 100 mL Tetrachlorkohlenstoff gelöst und unter Rühren zum sieden erhitzt. In der Siedehitze wurden portionsweise 170 mg (1,00 mmol) 2,2'-Azobis-2-methylpropannitril über 5 Stunden zugegeben und für eine weitere Stunde refluxiert. Nach Abkühlen auf Raumtemperatur wurde die Reaktionslösung filtriert und die Mutterlauge im Vakuum vom Lösungsmittel befreit. Durch chromatographische Reinigung wurde das gewünschte Produkt isoliert.

(logP: 4,12; MH⁺: 345; ¹H-NMR (400 MHz, DMSO, δ, ppm): 3,70 (s, 3H), 5,76 (s, 2H), 7,40-7,60 (m, 4 H.

### Beispiel Nr. XIII

### Synthese von Methyl-4-(2-chlorphenyl)-2-{[5-(trifluormethyl)-2H-tetrazol-2-yl]methyl}-1,3-thiazol-5-carboxylat

5,00 g (14,4 mmol) Methyl-2-(brommethyl)-4-(2-chlorphenyl)-1,3-thiazol-5-carboxylat wurden zu einer Lösung aus 10,00 g (18,7 mmol) Trifluormethyltetrazol-Natrium Salz in 100 mL Acetonitril gegeben. Anschließend wurden 600 mg (4,32 mmol) Kaliumcarbonat zur Reaktionslösung gegeben und für 6 Stunden bei 60 °C gerührt. Nach Abkühlen auf Raumtemperatur wurde die Reaktionslösung filtriert und die Mutterlauge im Vakuum vom Lösungsmittel befreit. Der Rückstand wurde mit 10 mL Wasser versetzt und gegen Essigester (3 x 20 mL) extrahiert. Die vereinigten organischen Phasen wurden über Natriumsulfat getrocknet. Das Lösungsmittel wurde im Vakuum entfernt und der Rückstand chromatographisch aufgereinigt. (logP: 3,60; MH⁺: 404; ¹H-NMR (400 MHz, DMSO, δ, ppm): 3,71 (s, 3H), 6,70 (s, 2H), 7,40-7,60 (m, 4 H).

### Beispiel Nr. X: (IV-2)

### Synthese von 4-(2-Chlorphenyl)-2-{[5-(trifluormethyl)-2H-tetrazol-2-yl]methyl}-1,3-thiazol-5-carbonsäure

3,60 g (8,91 mmol) Methyl-4-(2-chlorphenyl)-2-{[5-(trifluormethyl)-2H-tetrazol-2-yl]methyl}-1,3-thiazol-5-carboxylat wurden in 18 mL Dioxan und 6 mL Wasser gelöst und bei 0 °C tropfenweise mit 45%iger Natronlauge (10,06 mmol) versetzt. Die Reaktion wurde eine Stunde nachgerührt und auf RT erwärmt. Anschließend wurde das Ethanol unter vermindertem Druck entfernt und der Rückstand wurde mit Eiswasser (10 mL) versetzt. Die wässrige Phase wurde gegen Essigester extrahiert. Die organische Phase wurde verworfen. Anschließend wurde die wässrige Phase mit Salzsäure auf pH 3 eingestellt und nochmals gegen Essigester (3 x 20 mL) extrahiert. Die vereinigten organischen Phasen wurden über Natriumsulfat getrocknet. (logP: 2,63; MH⁺: 390; ¹H-NMR (400 MHz, DMSO, δ, ppm): 6,65 (s, 2H), 7,36-7,55 (m, 4 H.

### Synthese von Carbonsäuren der Formel IV-3

### Beispiel Nr. XV

### Synthese von Methyl 4-acetyl-1-(3-chlorpyridin-2-yl)-1H-pyrrol-2-carboxylat

5,0 g (15,6 mmol) Methyl 4-brom-1-(3-chlorpyridin-2-yl)-1H-pyrrol-2-carboxylat wurden unter Argon in THF gelöst und bei Raumtemperatur 8.2 g (21,9 mmol) (1-Ethoxyvinyl)-tributylstannan, 2,1g Lithiumchlorid und 0,9 g (0,8 mmol) Tetrakis(triphenylphosphin)palladium zugegeben und 48h unter Rückfluß gerührt. Anschließend wurde das Lösungsmittel abdestilliert, der Rückstand in 200 ml THF aufgenommen und in Gegenwart von 12 ml 2N Salzsäure weirere 24h bei RT gerührt. Das Lösungsmittel wurde erneut abdestilliert und der Rückstand in 120 ml Methyl-tert.-butylether gelöst und bei RT 1h mit 120 mL gesättigter Kaliumfluorid Lösung kräftig gerührt. Die organische Phase wurde abgetrennt und eingeengt. Durch chromatographische Reinigung wurde das gewünschte Produkt isoliert.

(logP: 1,74; MH⁺: 279; ¹H-NMR (400 MHz, DMSO, δ, ppm): 2,42 (s, 3H), 3,66 (s, 3H), 7,63-7,66 (m, 1H), 8,12 (s, 1H), 8,21 (d, 1H), 8,54 (d, 1H).

### Beispiel Nr. XVI

### Synthese von Methyl 4-(bromacetyl)-1-(3-chlorpyridin-2-yl)-1H-pyrrol-2-carboxylat

427 mg (1,48 mmol) Methyl 4-acetyl-1-(3-chlorpyridin-2-yl)-1H-pyrrol-2-carboxylat wurden in 12 ml Dioxan vorgelegt und bei RT 284 mg (1,78 mmol) Brom bei RT zugetropft. Anschließend ließ man 7h unter Rückfluß rühren. Das Lösungsmittel wurde im Vakuum abdestilliert wobei man ein Öl erhält, das ohne weitere Reinigung weiter umgesetzt wurde.

### (logP: 2,28; MH⁺: 358)

### Beispiel Nr. XVII

### Synthese von Methyl 1-(3-chlorpyridin-2-yl)-4-{[5-(trifluormethyl)-2H-tetrazol-2-yl]acetyl}-1H-pyrrol-2-carboxylat

890 mg (1,61 mmol) 5-(Trifluormethyl)-2H-tetrazol und 297 mg (2,14 mmol) Kaliumcarbonat wurden in 10 ml Acetonitril vorgelegt und 15 min zum Rückfluß erhitzt. 630 mg (1,07 mmol) Methyl 4-(bromacetyl)-1-(3-chlorpyridin-2-yl)-1H-pyrrol-2-carboxylat gelöst in 10 ml Acetonitril wurden zugegeben und die Reaktionsmischung 1h bei 70°C gerührt. Das Lösungsmittel wurde abdestilliert, der Rückstand in Wasser aufgenommen und zweimal mit Dichlormethan exthrahiert. Die vereinigten organischen Phasen wurden mit Natriumchlorid Lösung gewaschen, über Natriumsulfat getrocknet und eingeengt. Durch chromatographische Reinigung wurde das gewünschte Produkt isoliert.

(logP: 2,97; MH⁺: 415; ¹H-NMR (400 MHz, DMSO, δ, ppm): 3,69 (s, 3H), 6,65 (s, 2H), 7,57 (s, 1H), 7,67-7,70 (m, 1H), 8,25 (d, 1H), 8,36 (s, 1H), 8,57 (d, 1H).

### Beispiel Nr. IV-3:

### Synthese von 1-(3-Chlorpyridin-2-yl)-4-{[5-(trifluormethyl)-2H-tetrazol-2-yl]acetyl}-1H-pyrrole-2-carboxylic acid

130 mg (0,31 mmol) Methyl 1-(3-chlorpyridin-2-yl)-4-{[5-(trifluormethyl)-2H-tetrazol-2-yl]acetyl}-1H-pyrrol-2-carboxylat wurden in 3 ml Ethanol gelöst, mit 19 mg (0,47 mmol) 2N Natronlauge versetzt und 4h bei RT gerührt. Das Lösungsmittel wurde im Vakuum abdestilliert, der Rückstand in Wasser aufgenommern und einmal mit Methyl-tert.-butylether extrahiert. Anschließend wurde die wäßrige Phase mit 2N Salzsäure angesäuert und dreimal mit Essigester exthrahiert. Die vereinigten organischen Phasen wurden über Natriumsulfat getrocknet und eingeengt.

### (logP: 2,24; MH⁺: 401)

### Herstellungsbeispiele

Gemäß den oben beschriebenen Herstellverfahren lassen sich die Verbindungen der Formel (I) erhalten, beispielsweise die folgenden Verbindungen der Formel (I):

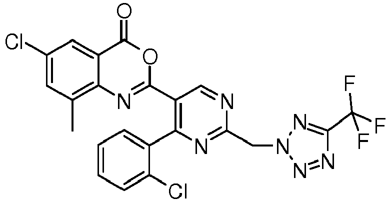

### Synthese von 6-Chlor-2-[4-(2-chlorphenyl)-2-{[5-(trifluormethyl)-2H-tetrazol-2-yl]methyl}pyrimidin-5-yl]-8-methyl-4H-3,1-benzoxazin-4-on

385 mg (1,30 mmol) 4-(2-Chlorphenyl)-2-{[5-(trifluormethyl)-2H-tetrazol-2-yl]methyl}pyrimidin-5-carbonsäure wurden bei RT in 5 mL Acetonitril vorgelegt und mit 0,27 mL (2,33 mmol) Lutidin versetzt. Die Lösung wurde anschließend unter Rühren auf 0 °C abgekühlt und 0,14 mL (1,78 mmol) Methansulfonsäurechlorid, gelöst in 2 mL Acetonitril, wurden tropfenweise addiert und 15 min. bei 0 °C nachgerührt. Diese Reaktionsmischung wurde nun mit einer Acetonitril-Suspension (3mL) aus 0,53 mL (4,54 mmol) Lutidin und 289 mg (1,56 mmol) 2-Amino-5-chlor-3-methylbenzoesäure portionsweise versetzt und 15 min. nachgerührt. Anschließend wurden 0,14 mL (1,78 mmol) Methansulfonsäurechlorid, gelöst in 2 mL Acetonitril, zugetropft. Der Reaktion wurde über Nacht gerührt und die Temperatur dabei auf RT erwärmt. Das Lösungsmittel wurde unter vermindertem Druck entfernt. Der Rückstand wurde anschließend auf Eiswasser gegossen um im Ultraschallbad verquirlt. Der Rückstand wurde abfiltriert, erneut in Wasser aufgenommen und nochmals im Ultraschallbad verquirlt. Der Rückstand wurde abgesaugt, mit Wasser nachgewaschen und im HV getrocknet. Es wurden 708 mg (88% d. Th.; gehalt 86%) des gewünschten Produktes isoliert. Eine weitere Aufreinung findet nicht statt.

logP: 5,16; MH⁺: 534; ¹H-NMR (400 MHz, DMSO-d₆, δ, ppm): 1,96 (s, 3H), 6,67 (s, 2H), 7,40-7,55 (m, 5H), 7,83 (m, 1H), 7,94 (m, 1H).

### Syntehese von N-[4-Chlor-2-methyl-6-(methylcarbamoyl)phenyl]-4-(2-chlorphenyl)-2-{[5-(trifluormethyl)-2H-tetrazol-2-yl]methyl}pyrimidin-5-carboxamid

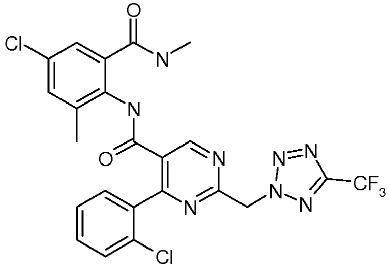

Es wurden 100 mg (0,18 mmol) 6-Chlor-2-[4-(2-chlorphenyl)-2-{[5-(trifluormethyl)-2H-tetrazol-2-yl]methyl}pyrimidin-5-yl]-8-methyl-4H-3,1-benzoxazin-4-on in 3 mL THF vorgelegt und bei RT mit 0,234 mL (0,46 mmol) einer 2 molaren Methylamin/THF Lösung versetzt. Anschließend wurde die Reaktion für 15 min bei 45 °C gerührt. Nach abkühlen wurde der Ansatz eingeengt und chromatographisch an Kieselgel mit Cyclohexan/Essigester aufgereinigt. Es wurden 48 mg (40% d. Th.; gehalt 89%) des gewünschten Produktes isoliert.

logP: 3,13; MH⁺: 566; ¹H-NMR (400 MHz, DMSO-d₆, δ, ppm): 2,05 (s, 3H), 2,69 (m, 3H), 6,60 (s, 2H), 7,35 (s, 1H), 7,40-7,55 (m, 4H), 8,32 (m, 1H); 9,13 (s, 1H).

Die folgenden Beispiele können auf analoge Art und Weise erhalten werden, wobei die Verbindungen der Formel (I) teils als Regioisomere vorliegen können. In der folgenden Tabelle sind in Bezug auf die NMR-Daten jeweils die chemischen Verschiebungen und die dazugehörigen Signalintensitäten angegeben, beispielsweise steht für Verbindung 1:
Signal 1
   10.272; 4.21; für 10.272 ppm (chemische Verschiebung), 4.21 (Signalintensität);
Signal 2
   9.899;0.35; für 9.899ppm (chemische Verschiebung), 0.35 (Signalintensität)

| NR | Structure | Log p | MH+ | NMR |
|---|---|---|---|---|
| 1 | 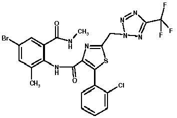 | 3,98 | 614 | (10.272;4.21),(9.899;0.35),(8.370;1.46),(8.360;1.45),(8.350;0.70),(7.642;0.44),(7.639;0.40),(7.637;0.39) ,(7.562;4.89),(7.542;3.41),(7.540;3.35),(7.528;2.10),(7.524;2.29),(7.510;3.13),(7.505;3.62),(7.499;4.18), (7.494;3.45),(7.479;1.98),(7.474;1.78),(7.460;3.04),(7.455;2.54),(7.440;1.89),(7.435;1.71),(7.407;2.18),( 7.404;2.16),(7.388;2.82),(7.385;2.65),(7.369;1.06),(7.366;1.01),(7.301;0.39),(6.706;12.44),(6.644;0.87), (6.598;0.53),(6.468;0.58),(4.040;0.62),(4.022;0.60),(3.380;0.46),(3.358;0.75),(3.321;2.64),(3.288;1167.9 7),(3.252;1.33),(3.228;0.60),(3.202;0.40),(2.890;0.35),(2.732;0.45),(2.695;0.57),(2.672;1.90),(2.668;2.4 1),(2.654;10.21),(2.642;10.01),(2.605;0.96),(2.593;0.96),(2.538;8.56),(2.521;7.60),(2.508;108.05),(2.50 3;210.37),(2.499;278.91),(2.494;197.59),(2.490;92.86),(2.407;0.65),(2.330;1.35),(2.326;1.84),(2.321;1.3 0),(2.316;0.73),(2.210;0.77),(2.158;1.30),(2.114;15.00),(2.095;1.07),(2.067;2.47),(2.049;1.34),(1.986;2. 65),(1.962;1.18),(1.293;0.71),(1.237;0.87),(1.193;0.77),(1.175;1.48),(1.157;0.87),(0.008;1.54),(-0.000;32.01),(-0.009;1.13) |
| 2 | 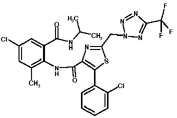 | 4,34 | 598 | (10.088;0.36),(8.190;1.14),(8.177;1.17),(7.554;1.44),(7.552;1.53),(7.541;1.84),(7.539;1.81),(7.512;1.24) ,(7.509;1.42),(7.499;1.65),(7.497;1.80),(7.471;0.93),(7.468;1.02),(7.458;1.54),(7.456;1.40),(7.445;1.14), (7.442;1.03),(7.435;1.75),(7.434;1.76),(7.431;2.03),(7.430;1.87),(7.401;1.25),(7.399;1.24),(7.396;0.35),( 7.389;1.88),(7.386;1.84),(7.376;0.82),(7.374;0.82),(7.323;1.98),(7.319;1.92),(6.705;7.83),(6.619;0.95),( 6.476;0.44),(3.887;0.52),(3.876;0.77),(3.863;0.75),(3.852;0.51),(3.344;455.59),(3.325;0.59),(3.320;0.58) ,(3.318;0.49),(2.884;1.07),(2.618;0.59),(2.615;0.85),(2.612;0.60),(2.542;4.85),(2.524;1.47),(2.521;1.89), (2.518;1.74),(2.509;44.47),(2.506;100.07),(2.503;137.84),(2.500;99.26),(2.497;44.89),(2.405;0.38),(2.39 0;0.64),(2.387;0.92),(2.384;0.66),(2.216;0.58),(2.153;0.45),(2.122;9.53),(2.102;0.72),(2.097;0.77),(2.07 7;1.50),(2.053;0.73),(1.918;0.43),(1.397;12.10),(1.292;0.37),(1.113;1.01),(1.102;1.00),(1.087;1.54),(1.0 |
| | | | | 76;1.58),(1.061;0.98),(1.051;0.98),(0.977;1.01),(0.966;13.50),(0.955;12.69),(0.005;0.81) |
| 3 | 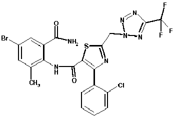 | 3,45 | 601 | (9.988;2.73),(9.866;0.46),(7.879;1.50),(7.715;0.51),(7.710;0.49),(7.617;0.56),(7.576;3.08),(7.546;4.65),( 7.541;3.66),(7.522;2.60),(7.517;2.94),(7.502;4.98),(7.498;5.10),(7.490;3.56),(7.454;1.56),(7.449;1.89),( 7.436;3.17),(7.431;2.65),(7.417;3.88),(7.412;3.31),(7.398;1.92),(7.378;0.82),(7.301;0.48),(7.026;0.62),( 7.005;0.54),(6.871;0.57),(6.718;0.65),(6.675;10.68),(6.628;0.48),(6.263;0.49),(5.742;8.23),(3.602;0.48), (3.567;0.54),(3.470;0.45),(3.453;0.55),(3.422;0.64),(3.404;0.74),(3.395;0.95),(3.288;1896.54),(3.265;32. 89),(3.228;0.78),(2.907;0.68),(2.695;0.52),(2.672;3.08),(2.668;4.03),(2.663;3.01),(2.639;0.49),(2.627;0. 56),(2.615;0.68),(2.597;0.77),(2.566;1.24),(2.538;6.31),(2.521;16.64),(2.508;226.17),(2.503;439.87),(2. 499;583.78),(2.494;412.79),(2.490;193.00),(2.405;5.02),(2.335;1.37),(2.330;2.69),(2.326;3.66),(2.321;2. 55),(2.204;1.79),(2.183;1.05),(2.102;15.00),(2.067;1.03),(2.048;1.11),(1.997;0.71),(1.986;0.55),(1.907;0 .63),(1.357;7.10),(1.246;2.61),(1.238;1.07),(1.175;0.49),(1.091;0.56),(0.891;0.84),(0.008;4.77),(-0.000;96.17),(-0.008;3.25) |
| 4 | 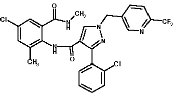 | 3,12 | 562 | (9.569;3.38),(9.561;2.92),(8.797;2.30),(8.554;2.92),(8.366;2.93),(8.284;1.17),(8.273;1.21),(8.237;2.95),( 8.208;1.46),(8.196;1.51),(8.085;1.28),(8.039;1.59),(8.035;1.63),(7.979;3.04),(7.958;1.93),(7.843;2.68),( 7.823;3.90),(7.695;1.85),(7.672;1.40),(7.543;1.58),(7.523;4.00),(7.513;1.73),(7.505;1.79),(7.498;2.15),( 7.491;2.74),(7.478;1.28),(7.471;1.44),(7.459;1.80),(7.454;2.63),(7.441;7.14),(7.439;6.11),(7.423;4.57),( 7.415;5.00),(7.408;4.25),(7.398;3.15),(7.380;3.15),(7.374;2.13),(7.361;3.29),(7.356;3.02),(7.343;1.92),( 7.335;3.37),(7.329;3.08),(7.321;4.15),(7.315;3.33),(5.654;6.11),(5.311;3.46),(5.282;3.39),(4.040;3.33),( 4.022;3.43),(3.290;1061.61),(2.684;2.12),(2.673;3.55),(2.668;3.02),(2.661;9.01),(2.652;14.18),(2.640;10 .86),(2.625;1.79),(2.613;1.67),(2.538;8.76),(2.521;7.47),(2.508;106.42),(2.504;208.63),(2.499;278.27),( 2.494;196.94),(2.490;92.43),(2.330;1.36),(2.326;1.80),(2.321;1.34),(2.214;2.59),(2.159;11.81),(2.129;14 .91),(2.109;2.80),(2.067;1.20),(2.049;1.49),(1.986;15.00),(1.193;4.21),(1.175;8.33),(1.157;4.16),(0.008; 1.13),(-0.000;25.35) |
| 5 | 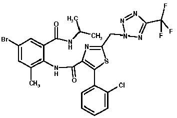 | 4,48 | 642 | (10.061;2.78),(8.151;1.25),(8.131;1.26),(7.548;3.69),(7.530;2.33),(7.527;2.37),(7.509;1.31),(7.504;1.54) ,(7.489;1.91),(7.485;2.10),(7.474;1.21),(7.470;1.16),(7.455;2.14),(7.451;1.81),(7.441;2.79),(7.436;3.59), (7.431;1.58),(7.403;1.61),(7.399;1.54),(7.384;1.99),(7.380;1.87),(7.365;0.78),(7.362;0.72),(6.688;8.65),( 6.462;0.64),(4.039;0.58),(3.899;0.66),(3.882;1.00),(3.866;0.93),(3.847;0.64),(3.398;0.96),(3.366;1.42),( 3.353;2.29),(3.293;3174.69),(3.271;32.58),(3.238;2.02),(3.230;1.25),(3.226;1.17),(3.174;0.60),(2.677;1. 57),(2.673;3.05),(2.668;4.09),(2.664;3.12),(2.584;0.58),(2.538;6.14),(2.522;16.14),(2.508;233.09),(2.50 4;458.49),(2.499;613.11),(2.495;434.14),(2.490;204.73),(2.335;1.49),(2.331;3.00),(2.326;3.94),(2.321;2. 81),(2.317;1.39),(2.151;0.57),(2.119;10.71),(2.095;1.00),(2.084;2.33),(2.067;0.88),(2.048;1.09),(1.986;2 .32),(1.399;3.01),(1.237;1.16),(1.193;0.75),(1.175;1.74),(1.168;1.76),(1.158;0.98),(1.092;0.93),(1.075;1. 09),(1.071;1.31),(1.054;0.99),(0.988;1.84),(0.977;15.00),(0.960;14.64),(0.891;0.85),(0.008;3.19),(-0.000;71.66),(-0.008;2.49) |
| 6 | 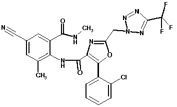 | 3,25 | 545 | (10.589;1.10),(8.533;0.87),(7.865;2.92),(7.830;3.67),(7.825;3.16),(7.804;0.43),(7.665;1.95),(7.661;2.04) ,(7.646;2.49),(7.642;2.62),(7.620;1.91),(7.618;2.00),(7.600;3.55),(7.597;3.41),(7.585;0.48),(7.575;1.73), (7.571;1.82),(7.557;2.55),(7.553;2.36),(7.537;1.44),(7.532;1.23),(7.478;1.93),(7.475;1.94),(7.459;2.66),( 7.456;2.62),(7.440;1.22),(7.437;1.17),(7.430;0.31),(6.625;11.62),(6.532;0.77),(6.358;0.84),(3.387;0.35), (3.376;0.37),(3.357;0.42),(3.290;663.56),(3.271;2.03),(3.256;0.62),(3.249;0.46),(2.858;4.27),(2.743;1.40 ),(2.731;1.83),(2.721;9.58),(2.709;9.44),(2.695;1.23),(2.683;1.00),(2.673;0.91),(2.668;1.08),(2.664;0.81) ,(2.591;0.42),(2.582;2.29),(2.570;2.19),(2.554;0.48),(2.538;4.69),(2.522;4.06),(2.508;55.84),(2.504;108. 12),(2.499;142.78),(2.495;100.13),(2.490;46.20),(2.331;0.70),(2.326;0.90),(2.322;0.63),(2.275;0.59),(2. 222;15.00),(2.209;1.70),(2.132;0.64),(2.099;1.22),(2.067;0.63),(2.049;0.69),(1.986;1.01),(1.293;0.36),(1 .238;0.50),(1.193;0.36),(1.175;0.62),(1.158;0.33),(0.008;0.70),(-0.000;14.39),(-0.008;0.42) |
| 7 | 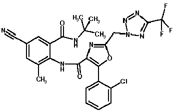 | 3,95 | 587 | (7.824;0.53),(7.725;0.63),(7.658;0.49),(7.655;0.54),(7.639;0.61),(7.635;0.63),(7.612;0.39),(7.595;0.82),( 7.592;0.77),(7.576;0.38),(7.573;0.38),(7.558;0.52),(7.554;0.46),(7.478;0.42),(7.474;0.40),(7.459;0.56),( 7.456;0.55),(6.624;2.30),(3.289;324.31),(2.914;0.37),(2.673;0.35),(2.668;0.47),(2.664;0.35),(2.538;2.32) ,(2.521;1.98),(2.508;27.37),(2.503;53.02),(2.499;70.11),(2.494;49.15),(2.490;22.66),(2.330;0.34),(2.326; 0.45),(2.321;0.32),(2.229;2.87),(2.091;0.56),(2.067;0.32),(2.049;0.36),(1.399;4.23),(1.368;2.51),(1.265; 0.37),(1.258;1.27),(1.211;0.94),(1.198;15.00),(-0.000;4.70) |
| 8 | 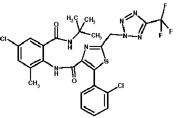 | 4,75 | 612 | (9.857;0.97),(7.722;0.97),(7.552;0.51),(7.550;0.55),(7.533;0.80),(7.530;0.77),(7.523;0.31),(7.502;0.49),( 7.498;0.64),(7.484;0.72),(7.479;0.89),(7.472;0.50),(7.458;0.75),(7.453;0.59),(7.438;0.53),(7.433;0.40),( 7.407;0.56),(7.403;0.59),(7.396;0.84),(7.388;1.18),(7.385;0.73),(7.250;0.90),(7.244;0.78),(6.675;2.99),( 6.643;0.48),(3.292;524.17),(3.269;7.71),(2.673;0.62),(2.668;0.81),(2.664;0.60),(2.659;0.31),(2.538;1.33) ,(2.521;3.70),(2.508;45.90),(2.504;88.96),(2.499;117.97),(2.495;83.54),(2.490;39.28),(2.330;0.53),(2.32 6;0.71),(2.321;0.50),(2.208;0.31),(2.157;0.59),(2.121;3.64),(2.067;0.41),(1.903;0.54),(1.399;1.91),(1.30 1;0.51),(1.285;2.65),(1.258;0.59),(1.237;0.42),(1.136;15.00),(-0.000;0.48) |
| 9 | 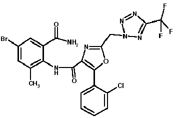 | 3,5 | 568 | (10.358;0.35),(8.002;1.93),(7.722;0.42),(7.718;0.42),(7.656;1.89),(7.654;2.03),(7.644;2.35),(7.641;2.36) ,(7.636;0.33),(7.632;0.57),(7.628;0.63),(7.617;5.45),(7.616;5.90),(7.613;4.99),(7.604;3.22),(7.602;3.02), (7.595;0.32),(7.592;0.43),(7.575;3.20),(7.571;2.86),(7.569;2.08),(7.566;1.69),(7.562;0.36),(7.556;2.16),( 7.554;2.04),(7.547;0.72),(7.543;1.80),(7.540;1.36),(7.528;0.35),(7.517;0.38),(7.513;0.43),(7.477;1.61),( 7.475;1.58),(7.464;2.37),(7.463;2.32),(7.452;1.13),(7.450;1.11),(7.308;0.37),(7.305;0.38),(7.117;1.48),( 6.613;10.31),(6.585;0.70),(6.343;0.44),(6.298;0.59),(3.400;0.35),(3.388;0.94),(3.376;1.22),(3.349;385.1 8),(3.321;0.42),(2.911;0.43),(2.618;0.64),(2.615;0.89),(2.612;0.64),(2.524;1.71),(2.521;2.11),(2.518;2.0 6),(2.510;47.18),(2.507;102.21),(2.503;141.07),(2.500;102.64),(2.497;47.08),(2.394;0.32),(2.391;0.66),( 2.388;0.93),(2.385;0.66),(2.382;0.32),(2.297;10.39),(2.215;0.95),(2.201;2.17),(2.169;13.50),(2.152;0.81) ,(2.102;2.09),(2.087;0.31),(2.077;0.84),(1.993;0.92),(1.990;0.48),(1.102;0.79),(1.091;1.60) |
| 10 | 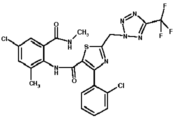 | 3,63 | 570 | (9.987;0.71),(8.380;0.66),(8.372;0.66),(7.572;0.41),(7.569;0.44),(7.541;0.50),(7.519;1.14),(7.506;1.35),( 7.501;0.63),(7.492;0.93),(7.489;0.94),(7.480;1.07),(7.477;1.11),(7.459;1.34),(7.455;1.43),(7.447;0.66),( 7.444;0.69),(7.434;1.01),(7.431;0.94),(7.421;0.74),(7.418;0.66),(7.409;0.87),(7.396;0.93),(7.381;0.51),( 7.377;0.37),(7.358;1.43),(7.355;1.37),(6.701;4.07),(3.346;323.35),(3.326;0.50),(3.323;0.53),(2.676;1.48) ,(2.671;3.58),(2.664;3.41),(2.621;0.33),(2.618;0.61),(2.615;0.84),(2.612;0.60),(2.609;0.30),(2.524;1.37), (2.521;1.75),(2.518;1.70),(2.509;40.48),(2.506;88.93),(2.503;122.93),(2.500;90.02),(2.497;41.16),(2.393 ;0.31),(2.390;0.63),(2.387;0.85),(2.384;0.73),(2.381;0.59),(2.372;6.77),(2.297;13.50),(2.212;1.02),(2.11 7;0.86),(2.107;6.31),(2.077;1.04),(1.910;0.28),(-0.000;8.27) |
| 11 | 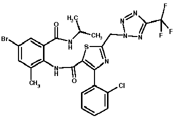 | 4,28 | 642 | (9.797;1.43),(8.189;0.59),(8.170;0.66),(7.697;0.37),(7.650;0.43),(7.610;0.62),(7.594;0.50),(7.556;1.43),( 7.518;0.95),(7.515;1.01),(7.498;1.41),(7.496;1.47),(7.465;0.98),(7.456;0.94),(7.450;0.99),(7.441;1.99),( 7.437;2.62),(7.418;0.96),(7.412;0.88),(7.407;0.95),(7.389;0.96),(7.368;0.47),(6.684;4.10),(3.940;0.43),( 3.923;0.64),(3.906;0.68),(3.890;0.43),(3.394;0.35),(3.292;371.20),(3.170;0.62),(2.695;0.33),(2.672;0.95) ,(2.668;1.27),(2.664;0.98),(2.538;5.69),(2.521;5.40),(2.508;72.92),(2.503;142.17),(2.499;188.71),(2.494; 133.99),(2.490;63.11),(2.330;0.85),(2.326;1.21),(2.321;0.89),(2.294;9.45),(2.212;0.68),(2.100;0.97),(2.0 72;5.68),(2.067;4.65),(2.048;0.95),(1.818;0.69),(1.293;0.46),(1.237;0.36),(1.179;15.00),(1.163;14.71),(1 .099;0.33),(1.091;0.54),(1.069;9.67),(1.052;9.55),(1.040;1.42),(1.018;0.42),(-0.000;20.17),(-0.009;0.74) |
| 12 | 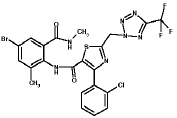 | 3,73 | 614 | (9.929;0.37),(8.322;1.01),(7.674;0.34),(7.612;0.38),(7.602;0.34),(7.573;1.91),(7.515;1.79),(7.495;3.21),( 7.473;3.66),(7.451;1.53),(7.446;1.56),(7.432;2.28),(7.427;2.02),(7.408;2.27),(7.390;1.48),(7.368;0.89),( 7.297;0.43),(7.284;0.35),(7.263;0.43),(7.233;0.30),(6.682;5.88),(6.490;0.33),(3.445;0.33),(3.432;0.32),( 3.382;0.49),(3.368;0.72),(3.288;1183.09),(3.248;1.31),(3.230;0.49),(3.202;0.33),(2.890;1.13),(2.732;1.0 7),(2.695;1.02),(2.672;6.92),(2.663;6.08),(2.622;0.63),(2.616;0.64),(2.600;0.61),(2.538;9.97),(2.521;8.9 4),(2.508;122.15),(2.503;238.04),(2.499;316.72),(2.494;224.10),(2.490;105.54),(2.406;0.53),(2.376;15.0 0),(2.330;1.52),(2.326;2.08),(2.321;1.40),(2.292;11.43),(2.209;0.86),(2.098;7.02),(2.067;6.38),(2.048;1. 99),(1.821;0.70),(1.356;0.48),(1.292;0.77),(1.236;0.59),(1.229;0.52),(1.159;0.49),(1.147;0.43),(0.008;1. 83),(-0.000;37.58),(-0.009;1.27) |
| 13 | 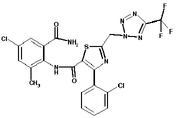 | 3,34 | 556 | (9.989;3.10),(7.871;1.72),(7.542;0.35),(7.522;2.40),(7.518;2.80),(7.503;5.54),(7.499;5.63),(7.449;3.85),( 7.444;3.82),(7.436;3.93),(7.431;3.53),(7.424;5.38),(7.418;6.72),(7.412;3.80),(7.399;2.05),(7.382;0.74),( 6.677;11.03),(6.629;0.30),(4.507;0.31),(4.057;1.13),(4.040;3.42),(4.022;3.40),(4.004;1.19),(3.353;0.46), (3.347;0.48),(3.286;568.48),(3.244;0.50),(2.673;0.88),(2.668;1.20),(2.663;0.92),(2.538;1.97),(2.521;5.52 ),(2.508;71.43),(2.503;137.28),(2.499;181.52),(2.494;128.45),(2.490;60.77),(2.405;1.41),(2.330;0.87),(2 .326;1.12),(2.321;0.82),(2.107;14.82),(2.067;1.33),(2.048;0.39),(1.986;15.00),(1.237;0.78),(1.193;4.22), (1.175;8.33),(1.157;4.16),(1.143;1.78),(0.008;1.53),(-0.000;30.10),(-0.008;1.13) |
| 14 | 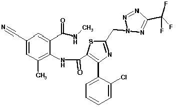 | 3,19 | 561 | (10.260;5.13),(10.169;1.17),(8.436;1.44),(8.399;0.59),(7.995;0.98),(7.894;0.95),(7.831;2.94),(7.800;1.12 ),(7.796;1.09),(7.765;4.29),(7.533;0.33),(7.514;2.84),(7.495;4.54),(7.473;1.89),(7.451;1.53),(7.446;1.74) ,(7.432;3.22),(7.427;2.70),(7.413;2.68),(7.408;3.27),(7.387;2.12),(7.369;0.76),(6.958;1.00),(6.689;14.82 ),(6.634;0.84),(3.515;0.31),(3.403;0.47),(3.337;1.34),(3.286;902.63),(3.248;0.53),(3.239;0.32),(2.857;5. 72),(2.747;0.33),(2.697;3.87),(2.685;13.99),(2.673;12.41),(2.634;0.96),(2.622;0.96),(2.582;3.28),(2.569; 3.29),(2.538;3.29),(2.521;8.00),(2.508;104.26),(2.503;200.60),(2.499;264.66),(2.494;187.38),(2.490;87. 91),(2.330;1.18),(2.326;1.58),(2.321;1.09),(2.277;4.17),(2.182;0.31),(2.143;15.00),(2.132;5.42),(2.115;0 .67),(2.067;1.18),(2.049;0.49),(1.986;0.78),(1.259;0.36),(1.238;0.84),(1.175;0.47),(1.143;0.77),(-0.000;31.49),(-0.008;1.13) |
| 15 | 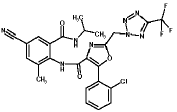 | 3,69 | 573 | (10.359;2.98),(10.099;0.48),(8.281;1.30),(8.262;1.35),(8.008;0.45),(7.885;0.45),(7.857;2.56),(7.784;2.64 ),(7.780;2.54),(7.765;0.46),(7.761;0.46),(7.657;1.41),(7.653;1.46),(7.638;1.76),(7.634;1.83),(7.613;1.15) ,(7.611;1.28),(7.593;2.56),(7.590;2.50),(7.574;1.37),(7.570;1.33),(7.556;1.79),(7.552;1.62),(7.535;0.94), (7.531;0.80),(7.504;0.44),(7.473;1.33),(7.469;1.29),(7.454;1.79),(7.451;1.74),(7.436;0.85),(7.432;0.73),( 7.004;0.46),(6.624;8.55),(6.364;0.57),(3.958;0.71),(3.942;1.07),(3.923;1.10),(3.907;0.74),(3.376;0.35),( 3.364;0.40),(3.347;0.48),(3.287;471.80),(3.248;0.38),(3.244;0.33),(2.878;1.70),(2.673;0.69),(2.668;0.87) ,(2.663;0.67),(2.576;0.51),(2.538;1.47),(2.508;50.27),(2.503;97.02),(2.499;128.26),(2.494;90.84),(2.490; 42.88),(2.330;0.59),(2.326;0.80),(2.321;0.58),(2.286;1.87),(2.230;10.81),(2.214;1.02),(2.128;1.78),(2.06 7;0.60),(1.986;0.35),(1.237;0.36),(1.120;1.69),(1.103;1.70),(1.082;2.79),(1.074;0.72),(1.066;2.78),(1.05 0;2.44),(1.043;15.00),(1.034;2.95),(1.026;14.66),(-0.000;12.20),(-0.008;0.42) |
| 16 | 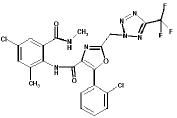 | 3,65 | 554 | (10.219;4.04),(10.143;0.33),(8.400;1.47),(8.389;1.48),(7.654;1.85),(7.650;2.01),(7.635;2.48),(7.631;2.54 ),(7.614;1.92),(7.611;1.97),(7.594;3.66),(7.591;3.64),(7.569;2.04),(7.565;2.04),(7.550;2.82),(7.546;2.63) ,(7.530;1.60),(7.526;1.33),(7.470;4.60),(7.464;3.64),(7.455;3.00),(7.451;2.67),(7.436;1.22),(7.433;1.16), (7.407;3.65),(7.401;3.05),(6.616;11.43),(6.573;0.65),(6.532;0.37),(6.352;0.69),(3.414;0.34),(3.389;0.44) ,(3.344;0.84),(3.341;0.83),(3.287;612.72),(2.726;0.40),(2.696;10.02),(2.685;10.00),(2.673;1.55),(2.668;1 .39),(2.663;1.04),(2.620;0.78),(2.608;0.73),(2.572;0.72),(2.538;2.21),(2.521;5.51),(2.508;68.34),(2.503; 130.43),(2.499;171.28),(2.494;120.33),(2.490;56.02),(2.330;0.81),(2.326;1.06),(2.321;0.75),(2.226;0.87) ,(2.215;0.49),(2.177;15.00),(2.164;1.47),(2.110;0.38),(2.067;0.72),(2.029;0.84),(1.986;0.30),(1.238;0.49 ),(-0.000;18.38),(-0.008;0.61) |
| 17 | 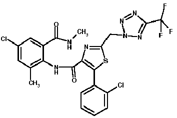 | 3,73 | 589 | (10.154;3.08),(10.099;0.53),(8.293;1.21),(8.274;1.17),(8.003;0.40),(7.889;0.42),(7.824;2.25),(7.766;0.53 ),(7.761;0.59),(7.747;2.58),(7.743;2.34),(7.544;0.31),(7.518;1.29),(7.514;1.39),(7.498;2.27),(7.495;2.65) ,(7.489;1.20),(7.475;1.44),(7.470;1.60),(7.455;0.90),(7.450;1.04),(7.437;2.00),(7.432;1.51),(7.418;1.53), (7.410;1.69),(7.405;1.45),(7.391;1.44),(7.387;1.32),(7.372;0.47),(7.276;0.33),(7.003;0.45),(6.692;8.48),( 6.633;0.54),(3.947;0.67),(3.930;1.00),(3.912;1.00),(3.895;0.69),(3.880;0.32),(3.359;0.47),(3.349;0.54),( 3.340;0.68),(3.334;0.76),(3.288;465.72),(3.265;0.90),(3.250;0.42),(2.878;1.61),(2.677;0.37),(2.673;0.66) |
| | | | | ,(2.668;0.84),(2.664;0.67),(2.538;1.40),(2.521;3.82),(2.508;48.03),(2.503;92.27),(2.499;121.60),(2.494;8 5.90),(2.490;40.32),(2.330;0.55),(2.326;0.77),(2.321;0.55),(2.286;1.81),(2.120;10.16),(2.067;0.57),(2.04 5;0.81),(1.399;0.57),(1.237;0.37),(1.220;0.59),(1.198;0.57),(1.181;0.58),(1.145;0.67),(1.120;1.71),(1.10 3;1.93),(1.085;14.93),(1.069;15.00),(1.057;1.61),(1.002;0.80),(0.986;0.80),(0.008;0.52),(-0.000;9.87),(-0.008;0.35) |
| 18 | 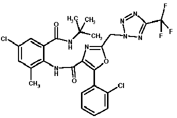 | 4,46 | 596 | (9.792;1.00),(7.806;0.38),(7.680;0.95),(7.646;0.64),(7.643;0.52),(7.627;0.79),(7.624;0.68),(7.612;0.54),( 7.609;0.53),(7.592;0.94),(7.589;0.94),(7.573;0.50),(7.569;0.50),(7.555;0.77),(7.535;0.54),(7.531;0.43),( 7.477;0.54),(7.473;0.51),(7.458;0.65),(7.455;0.62),(7.439;0.41),(7.432;0.81),(7.427;0.84),(7.272;0.86),( 7.267;0.81),(6.656;1.08),(6.618;2.85),(3.351;0.38),(3.339;0.53),(3.326;0.78),(3.287;405.26),(3.240;0.38) ,(2.673;0.57),(2.668;0.82),(2.664;0.56),(2.538;1.20),(2.521;3.46),(2.508;45.09),(2.503;86.46),(2.499;113 .87),(2.494;80.41),(2.490;38.03),(2.335;0.34),(2.330;0.58),(2.326;0.79),(2.321;0.57),(2.221;0.35),(2.207 ;0.37),(2.183;3.52),(2.162;0.32),(2.084;1.27),(2.067;0.37),(1.976;0.31),(1.399;7.60),(1.323;0.87),(1.281; 1.38),(1.258;0.82),(1.237;0.65),(1.182;1.48),(1.169;15.00),(0.008;0.63),(-0.000;12.07),(-0.008;0.46) |
| 19 | 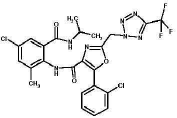 | 4,11 | 582 | (9.980;2.99),(8.130;1.34),(8.111;1.33),(7.644;1.40),(7.640;1.47),(7.625;1.77),(7.621;1.89),(7.606;1.25),( 7.588;2.67),(7.585;2.67),(7.568;1.46),(7.564;1.58),(7.550;1.91),(7.546;1.94),(7.530;1.18),(7.525;0.97),( 7.470;1.48),(7.466;1.50),(7.458;2.34),(7.451;3.95),(7.432;0.88),(7.429;0.87),(7.343;2.45),(7.337;2.39),( 6.613;8.21),(6.569;0.48),(6.358;0.54),(3.936;0.65),(3.920;0.97),(3.902;0.99),(3.884;0.62),(3.359;0.33),( 3.337;0.78),(3.286;821.35),(3.264;3.24),(3.243;1.02),(3.232;0.64),(3.217;0.47),(3.209;0.45),(3.181;0.33) ,(2.878;0.52),(2.672;1.20),(2.668;1.46),(2.663;1.13),(2.566;0.50),(2.538;2.18),(2.508;88.43),(2.503;172. 72),(2.499;230.42),(2.494;164.79),(2.490;78.91),(2.421;0.34),(2.404;0.40),(2.334;0.69),(2.330;1.20),(2. 326;1.60),(2.321;1.18),(2.218;0.73),(2.182;10.82),(2.165;1.05),(2.115;0.59),(2.067;0.80),(2.048;0.35),(2 .002;0.64),(1.986;0.77),(1.237;0.58),(1.175;0.42),(1.143;1.24),(1.130;0.61),(1.119;0.60),(1.113;0.62),(1. 103;0.52),(1.064;1.06),(1.047;0.99),(1.019;1.95),(1.010;15.00),(0.993;14.69),(0.008;1.51),(-0.000;31.10),(-0.008;1.13) |
| 20 | 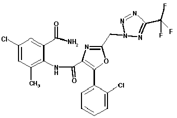 | 3,45 | 540 | (10.319;3.92),(10.272;0.43),(7.945;1.77),(7.658;2.01),(7.654;2.21),(7.639;2.68),(7.635;2.80),(7.613;1.88 ),(7.610;2.08),(7.593;3.63),(7.590;3.72),(7.569;2.87),(7.565;3.49),(7.551;3.28),(7.547;2.87),(7.531;1.73) ,(7.527;1.50),(7.474;4.37),(7.468;4.27),(7.454;6.22),(7.448;2.86),(7.440;1.60),(7.436;1.46),(7.416;0.31), (6.600;11.06),(6.573;0.43),(6.524;0.63),(6.336;0.84),(4.058;0.44),(4.040;1.23),(4.022;1.24),(4.004;0.38) ,(3.376;0.35),(3.288;811.89),(3.263;2.05),(3.236;0.45),(3.226;0.38),(2.908;2.63),(2.673;1.00),(2.668;1.3 3),(2.664;1.00),(2.562;0.76),(2.538;2.22),(2.521;5.77),(2.508;78.08),(2.503;151.32),(2.499;200.22),(2.4 94;141.72),(2.490;66.55),(2.331;0.99),(2.326;1.33),(2.321;0.95),(2.220;0.57),(2.211;0.94),(2.174;15.00), (2.157;1.59),(2.109;0.87),(2.067;0.43),(2.049;0.34),(2.005;0.46),(1.986;5.36),(1.399;0.40),(1.260;0.34),( 1.237;0.70),(1.193;1.55),(1.175;3.07),(1.157;1.52),(-0.000;8.81) |
| 21 | 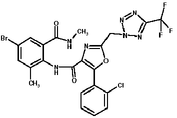 | 3,72 | 598 | (10.220;4.05),(10.145;0.36),(8.404;1.53),(8.393;1.42),(7.667;0.62),(7.653;1.92),(7.649;2.05),(7.634;2.56 ),(7.630;2.57),(7.614;2.16),(7.611;2.36),(7.595;6.70),(7.591;4.84),(7.569;2.02),(7.565;2.02),(7.550;2.78) ,(7.546;2.51),(7.530;4.78),(7.526;3.86),(7.473;1.99),(7.470;1.88),(7.455;2.69),(7.452;2.48),(7.436;1.17), (7.433;1.10),(6.614;11.44),(6.570;0.45),(6.532;0.52),(6.350;0.72),(5.742;1.36),(3.457;0.34),(3.444;0.40) ,(3.433;0.39),(3.422;0.51),(3.406;0.47),(3.372;0.78),(3.361;0.98),(3.350;1.26),(3.291;1150.47),(3.263;1. 94),(3.245;0.46),(3.240;0.43),(2.694;10.26),(2.683;10.38),(2.668;1.93),(2.664;1.38),(2.618;0.63),(2.607; 0.62),(2.588;0.41),(2.571;0.77),(2.538;2.70),(2.521;6.72),(2.508;88.67),(2.504;171.37),(2.499;226.67),( 2.495;160.36),(2.490;75.44),(2.330;1.14),(2.326;1.38),(2.322;0.96),(2.222;0.58),(2.210;0.65),(2.173;15. 00),(2.161;1.60),(2.105;0.80),(2.067;1.58),(2.049;0.34),(2.024;0.59),(1.399;0.33),(1.237;0.69),(1.143;0. 50),(0.891;0.31),(-0.000;5.52) |
| 22 | 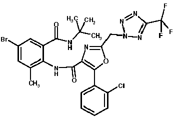 | 4,52 | 640 | (9.788;0.98),(7.689;1.04),(7.672;0.31),(7.669;0.32),(7.646;0.59),(7.642;0.54),(7.627;0.81),(7.623;0.71),( 7.609;0.56),(7.593;0.96),(7.589;0.99),(7.573;0.60),(7.569;0.78),(7.564;0.88),(7.560;0.94),(7.555;0.95),( 7.551;0.77),(7.535;0.52),(7.477;0.48),(7.473;0.49),(7.458;0.68),(7.455;0.70),(7.439;0.30),(7.436;0.30),( 7.393;0.85),(7.387;0.82),(6.655;0.69),(6.616;2.82),(3.290;390.54),(2.914;2.43),(2.673;0.47),(2.668;0.65) ,(2.664;0.52),(2.538;1.09),(2.521;2.74),(2.508;36.60),(2.504;70.94),(2.499;94.11),(2.494;66.79),(2.490;3 1.47),(2.330;0.49),(2.326;0.60),(2.321;0.46),(2.316;0.46),(2.217;0.31),(2.180;3.53),(2.159;0.31),(2.079; 0.85),(2.067;0.60),(1.655;1.38),(1.399;9.88),(1.336;0.32),(1.323;1.12),(1.284;1.36),(1.258;7.15),(1.247; 0.43),(1.237;0.50),(1.181;1.56),(1.169;15.00),(0.008;0.43),(-0.000;9.05),(-0.008;0.32) |
| 23 | 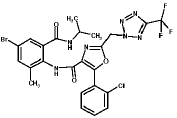 | 4,21 | 626 | (9.984;2.89),(8.141;1.34),(8.122;1.34),(7.644;1.34),(7.640;1.49),(7.624;1.79),(7.621;1.87),(7.605;1.39),( 7.588;4.43),(7.585;4.98),(7.568;1.48),(7.564;1.51),(7.550;1.92),(7.546;1.78),(7.530;1.04),(7.525;0.90),( 7.466;3.66),(7.460;2.64),(7.451;2.14),(7.448;1.83),(7.432;0.97),(7.429;0.92),(6.613;8.29),(6.567;0.48),( 6.514;0.39),(6.357;0.52),(3.952;0.31),(3.935;0.67),(3.919;1.05),(3.902;0.97),(3.884;0.64),(3.355;0.35),( 3.286;656.24),(3.240;0.57),(3.230;0.45),(3.223;0.42),(3.216;0.38),(3.210;0.37),(2.877;1.93),(2.677;0.58) ,(2.672;1.04),(2.668;1.29),(2.663;0.92),(2.566;0.40),(2.538;2.12),(2.521;5.52),(2.508;76.68),(2.503;148. 33),(2.499;196.43),(2.494;139.48),(2.490;66.19),(2.421;0.35),(2.335;0.66),(2.330;1.00),(2.326;1.31),(2. 321;0.91),(2.215;0.71),(2.179;10.77),(2.162;1.07),(2.115;0.67),(2.067;1.64),(2.049;0.40),(1.997;0.58),(1 .440;0.31),(1.423;0.30),(1.399;2.60),(1.238;0.54),(1.143;1.66),(1.130;0.79),(1.119;1.96),(1.114;0.96),(1. 103;1.87),(1.067;1.04),(1.050;0.94),(1.020;2.05),(1.011;15.00),(0.994;14.70),(0.008;1.51),(-0.000;30.14),(-0.009;1.12) |
| 24 | 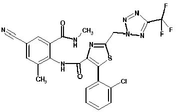 | 3,46 | 560 | (10.654;3.64),(10.169;0.62),(8.497;1.42),(8.485;1.47),(8.410;0.31),(8.398;0.33),(7.996;0.58),(7.992;0.56 ),(7.893;0.56),(7.826;3.47),(7.796;4.36),(7.570;2.00),(7.567;2.07),(7.550;3.17),(7.547;3.10),(7.539;1.98) ,(7.535;2.12),(7.520;2.60),(7.516;2.82),(7.486;1.35),(7.482;1.43),(7.467;2.60),(7.463;2.12),(7.448;1.70), (7.443;1.40),(7.425;0.41),(7.413;1.96),(7.410;1.96),(7.394;2.57),(7.391;2.46),(7.376;1.02),(7.373;0.91),( 6.960;0.61),(6.718;12.51),(6.477;0.51),(4.058;0.38),(4.040;1.03),(4.022;1.11),(4.004;0.39),(3.440;0.35), |
| | | | | (3.426;0.30),(3.400;0.37),(3.375;0.63),(3.346;0.97),(3.328;1.75),(3.289;892.22),(3.254;0.94),(2.857;3.05 ),(2.742;1.05),(2.731;1.07),(2.697;2.06),(2.680;10.17),(2.668;11.07),(2.650;0.58),(2.582;1.83),(2.569;1. 85),(2.538;2.40),(2.521;6.13),(2.508;82.52),(2.504;159.84),(2.499;211.72),(2.494;149.90),(2.490;70.75), (2.330;0.97),(2.326;1.27),(2.321;0.98),(2.277;2.42),(2.244;0.32),(2.159;15.00),(2.141;1.14),(2.132;2.52) ,(2.098;1.40),(2.067;0.38),(2.049;0.35),(1.986;4.67),(1.238;0.45),(1.193;1.32),(1.175;2.65),(1.157;1.28), (-0.000;7.74) |
| 25 | 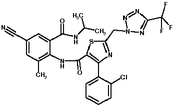 | 3,7 | 589 | (10.154;3.08),(10.099;0.53),(8.293;1.21),(8.274;1.17),(8.003;0.40),(7.889;0.42),(7.824;2.25),(7.766;0.53 ),(7.761;0.59),(7.747;2.58),(7.743;2.34),(7.544;0.31),(7.518;1.29),(7.514;1.39),(7.498;2.27),(7.495;2.65) ,(7.489;1.20),(7.475;1.44),(7.470;1.60),(7.455;0.90),(7.450;1.04),(7.437;2.00),(7.432;1.51),(7.418;1.53), (7.410;1.69),(7.405;1.45),(7.391;1.44),(7.387;1.32),(7.372;0.47),(7.276;0.33),(7.003;0.45),(6.692;8.48),( 6.633;0.54),(3.947;0.67),(3.930;1.00),(3.912;1.00),(3.895;0.69),(3.880;0.32),(3.359;0.47),(3.349;0.54),( 3.340;0.68),(3.334;0.76),(3.288;465.72),(3.265;0.90),(3.250;0.42),(2.878;1.61),(2.677;0.37),(2.673;0.66) ,(2.668;0.84),(2.664;0.67),(2.538;1.40),(2.521;3.82),(2.508;48.03),(2.503;92.27),(2.499;121.60),(2.494;8 5.90),(2.490;40.32),(2.330;0.55),(2.326;0.77),(2.321;0.55),(2.286;1.81),(2.120;10.16),(2.067;0.57),(2.04 5;0.81),(1.399;0.57),(1.237;0.37),(1.220;0.59),(1.198;0.57),(1.181;0.58),(1.145;0.67),(1.120;1.71),(1.10 3;1.93),(1.085;14.93),(1.069;15.00),(1.057;1.61),(1.002;0.80),(0.986;0.80),(0.008;0.52),(-0.000;9.87),(-0.008;0.35) |
| 26 | 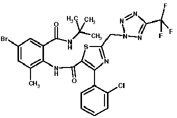 | 4,69 | 656 | (9.644;0.89),(7.750;0.80),(7.531;1.06),(7.527;1.04),(7.511;0.69),(7.508;0.67),(7.488;0.33),(7.483;0.38),( 7.470;0.53),(7.465;0.78),(7.447;0.66),(7.442;0.44),(7.428;0.45),(7.423;0.33),(7.415;0.50),(7.411;0.51),( 7.392;1.21),(7.386;0.80),(6.690;2.52),(3.323;0.30),(3.285;179.92),(2.668;0.39),(2.538;0.61),(2.521;1.67) ,(2.508;22.60),(2.503;43.85),(2.499;58.18),(2.494;41.34),(2.490;19.66),(2.326;0.37),(2.048;3.46),(1.399; 3.25),(1.257;15.00),(1.147;0.46),(-0.000;4.74) |
| 27 | 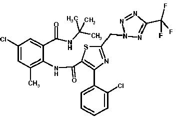 | 4,59 | 612 | (9.643;0.80),(7.736;0.80),(7.528;0.47),(7.509;0.72),(7.485;0.40),(7.465;0.81),(7.448;0.60),(7.428;0.40),( 7.412;0.55),(7.396;1.19),(7.272;0.79),(6.690;2.34),(3.370;0.42),(3.292;755.22),(2.673;0.72),(2.668;0.98) ,(2.664;0.71),(2.538;1.56),(2.521;3.96),(2.508;56.59),(2.504;109.81),(2.499;145.54),(2.494;103.80),(2.4 90;49.44),(2.330;0.70),(2.326;0.94),(2.321;0.68),(2.067;1.65),(2.051;4.07),(1.256;15.00),(1.237;0.91),(-0.000;3.07) |
| 28 | 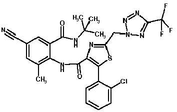 | 4,29 | 603 | (10.188;1.04),(7.891;0.95),(7.795;0.79),(7.792;0.84),(7.698;0.90),(7.694;0.83),(7.558;0.45),(7.555;0.48) ,(7.538;0.75),(7.535;0.75),(7.520;0.45),(7.516;0.50),(7.501;0.61),(7.497;0.70),(7.483;0.35),(7.479;0.35), (7.465;0.64),(7.460;0.49),(7.445;0.41),(7.440;0.31),(7.411;0.48),(7.408;0.48),(7.393;0.64),(7.390;0.60),( 6.689;3.03),(3.288;303.81),(2.914;3.10),(2.672;0.41),(2.668;0.54),(2.663;0.40),(2.538;0.93),(2.521;2.32) ,(2.508;30.06),(2.503;57.99),(2.499;76.67),(2.494;54.35),(2.490;25.58),(2.330;0.36),(2.326;0.48),(2.321; 0.33),(2.166;3.66),(2.067;0.34),(1.399;2.40),(1.258;9.18),(1.169;15.00),(1.107;0.45),(-0.000;4.49) |
| 29 | 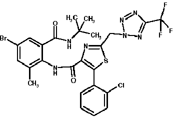 | 4,83 | 656 | (9.855;0.97),(7.735;0.93),(7.553;0.46),(7.550;0.48),(7.530;1.35),(7.523;0.82),(7.503;0.43),(7.498;0.46),( 7.484;0.60),(7.480;0.75),(7.472;0.36),(7.458;0.66),(7.453;0.46),(7.438;0.41),(7.406;0.50),(7.403;0.46),( 7.388;0.64),(7.384;0.60),(7.369;1.03),(7.365;0.88),(6.674;2.91),(3.289;260.58),(3.266;1.22),(2.890;0.32) ,(2.668;0.42),(2.538;0.70),(2.521;1.86),(2.508;23.16),(2.503;44.03),(2.499;57.70),(2.494;40.55),(2.490;1 8.90),(2.326;0.35),(2.118;3.61),(2.067;0.45),(1.137;15.00),(-0.000;1.72) |
| 30 | 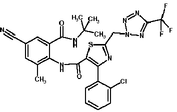 | 4,07 | 603 | (9.995;1.09),(7.892;0.90),(7.798;0.76),(7.795;0.79),(7.712;0.87),(7.708;0.81),(7.530;0.41),(7.527;0.44),( 7.510;0.65),(7.507;0.69),(7.496;0.40),(7.492;0.40),(7.478;0.58),(7.473;0.67),(7.461;0.31),(7.447;0.64),( 7.442;0.46),(7.428;0.44),(7.422;0.36),(7.418;0.53),(7.414;0.51),(7.399;0.53),(7.396;0.48),(6.697;3.01),( 3.289;191.50),(2.668;0.35),(2.538;0.60),(2.521;1.48),(2.508;19.64),(2.503;37.90),(2.499;50.12),(2.494;3 5.46),(2.490;16.65),(2.326;0.31),(2.095;3.50),(2.084;0.88),(1.274;15.00),(1.107;0.34),(0.008;0.31),(-0.000;6.22) |
| 31 | 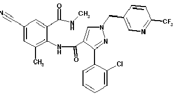 | 2,78 ; 2,83 | 553 | (9.907;3.99),(9.902;4.46),(8.798;2.03),(8.594;5.64),(8.434;1.02),(8.423;1.09),(8.372;3.44),(8.251;7.12),( 8.064;0.99),(8.040;1.43),(7.981;2.58),(7.960;1.65),(7.845;2.19),(7.824;4.36),(7.818;2.90),(7.808;2.98),( 7.756;2.74),(7.752;2.64),(7.741;3.23),(7.737;2.81),(7.697;1.53),(7.680;1.20),(7.544;1.00),(7.528;2.76),( 7.517;1.38),(7.511;1.32),(7.502;1.64),(7.496;1.94),(7.481;0.88),(7.476;1.43),(7.469;1.17),(7.460;1.76),( 7.455;4.07),(7.448;4.23),(7.437;2.52),(7.429;2.65),(7.425;1.53),(7.412;1.93),(7.406;2.19),(7.397;0.90),( 7.384;2.02),(7.379;1.35),(7.366;3.02),(7.361;2.20),(7.348;1.49),(7.344;1.13),(5.661;5.52),(5.360;0.85),( 5.321;2.82),(5.291;2.72),(5.252;0.89),(3.394;0.79),(3.331;2.89),(3.289;1820.16),(3.241;1.54),(3.077;1.0 3),(2.678;8.35),(2.668;15.00),(2.657;9.02),(2.610;0.60),(2.576;0.87),(2.538;4.94),(2.521;12.93),(2.508;1 67.56),(2.503;322.86),(2.499;426.89),(2.494;301.29),(2.490;141.85),(2.330;2.07),(2.326;2.69),(2.321;1. 95),(2.201;10.77),(2.175;12.79),(2.067;1.01),(2.049;0.60),(1.237;1.07),(1.107;3.59),(0.008;2.21),(-0.000;42.38),(-0.008;1.53) |
| 32 | 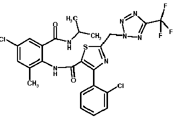 | 4,15 | 598 | (9.805;2.15),(8.191;0.89),(8.172;0.92),(7.521;0.98),(7.517;1.20),(7.501;1.72),(7.498;1.88),(7.490;0.78),( 7.486;0.81),(7.468;1.16),(7.457;0.80),(7.452;0.89),(7.43 8;1.94),(7.433;2.46),(7.426;1.73),(7.419;1.48),( 7.413;1.12),(7.409;1.15),(7.405;1.14),(7.391;1.07),(7.387;1.02),(7.369;0.39),(7.320;2.29),(7.314;2.09),( 6.688;6.51),(3.942;0.55),(3.925;0.83),(3.907;0.83),(3.890;0.55),(3.307;1395.40),(3.286;2.93),(3.284;2.9 9),(3.261;0.57),(3.251;0.48),(2.678;0.40),(2.674;0.92),(2.669;1.32),(2.664;0.97),(2.660;0.39),(2.539;1.8 2),(2.523;2.96),(2.518;4.47),(2.509;69.12),(2.505;138.11),(2.500;189.23),(2.496;128.68),(2.491;60.03),( 2.336;0.43),(2.331;0.97),(2.327;1.33),(2.322;0.94),(2.318;0.45),(2.076;8.52),(2.049;0.37),(1.236;0.48),( 1.068;15.00),(1.051;14.92),(-0.000;5.23) |
| 33 | 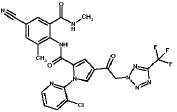 | 2,75 | 572 | (10.327;4.85),(8.523;3.11),(8.519;3.40),(8.511;3.41),(8.508;3.34),(8.373;4.68),(8.369;4.70),(8.358;0.92) ,(8.346;1.84),(8.335;1.85),(8.160;2.69),(8.156;2.75),(8.139;2.90),(8.136;2.84),(7.839;3.72),(7.749;4.03), (7.745;3.90),(7.699;4.39),(7.695;4.43),(7.608;2.71),(7.596;2.64),(7.588;2.60),(7.576;2.74),(6.615;13.56) ,(4.095;0.52),(4.039;0.54),(4.021;0.61),(3.341;2.34),(3.304;2412.98),(3.281;8.17),(3.263;1.33),(3.241;0. 61),(3.232;0.67),(2.858;7.44),(2.716;1.07),(2.704;1.18),(2.684;11.00),(2.673;12.46),(2.664;3.07),(2.660; 1.45),(2.581;3.88),(2.568;3.91),(2.539;5.04),(2.522;8.01),(2.518;11.83),(2.509;157.46),(2.504;311.75),( 2.500;425.12),(2.495;294.21),(2.491;140.21),(2.336;1.02),(2.331;2.05),(2.327;2.89),(2.322;2.02),(2.273; 1.38),(2.209;16.00),(2.069;2.95),(2.049;0.66),(1.987;2.63),(1.258;0.50),(1.237;2.61),( 1.193;0.97),( 1.175 ;1.70),(1.157;1.00),(1.127;0.73),(1.113;1.96),(1.109;1.50),(1.098;1.95),(1.091;0.79),(0.008;2.33),(-0.000;72.01),(-0.008;2.37) |
| 34 | 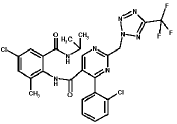 | 3,91 | 593 | (10.259;0.31),(10.240;3.40),(9.133;6.05),(8.266;1.50),(8.246;1.50),(7.517;1.05),(7.500;2.72),(7.489;1.52 ),(7.483;1.51),(7.473;1.54),(7.467;2.01),(7.461;1.17),(7.455;1.62),(7.448;1.85),(7.441;3.23),(7.432;2.84) ,(7.427;3.73),(7.420;3.53),(7.397;0.59),(7.393;0.54),(7.325;2.90),(7.319;2.75),(6.605;8.47),(6.398;0.58), (3.973;0.65),(3.956;1.00),(3.939;1.00),(3.921;0.65),(3.306;374.18),(3.210;0.42),(2.674;0.48),(2.669;0.61 ),(2.664;0.48),(2.539;1.46),(2.504;69.08),(2.500;86.17),(2.496;62.29),(2.425;0.39),(2.327;0.69),(2.322;0 .54),(2.069;0.93),(2.031;11.83),(2.012;0.73),(1.987;0.32),(1.398;1.91),(1.237;0.35),(1.087;14.95),(1.071 ;15.00),(1.035;0.45),(1.018;0.38),(-0.000;6.72) |
| 35 | 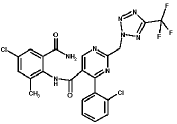 | 3,01 | 551 | (10.344;0.30),(10.334;0.33),(10.305;4.35),(9.179;0.35),(9.142;7.79),(7.840;2.15),(7.526;1.57),(7.513;1.5 5),(7.507;2.75),(7.502;2.79),(7.489;1.75),(7.483;2.35),(7.474;3.04),(7.467;4.83),(7.462;6.42),(7.457;5.4 7),(7.451;5.07),(7.445;4.32),(7.439;4.51),(7.434;5.23),(7.414;0.86),(7.393;4.11),(7.387;3.31),(6.603;10. 93),(6.394;0.53),(3.530;0.31),(3.471;0.50),(3.308;991.99),(3.238;1.02),(3.192;0.41),(3.128;0.30),(2.731; 0.31),(2.694;0.30),(2.673;1.07),(2.669;1.34),(2.609;0.47),(2.592;0.55),(2.539;8.38),(2.508; 80.27),(2.504 ;141.87),(2.500;179.30),(2.496;126.41),(2.412;0.35),(2.331;0.96),(2.326;1.23),(2.322;0.94),(2.097;0.40), (2.084;3.55),(2.069;3.08),(2.058;15.00),(1.987;0.50),(1.907;0.30),(1.398;10.46),(1.292;0.35),(1.235;0.65 ),(1.107;0.30),(0.890;0.43),(-0.000;2.02) |
| 36 | 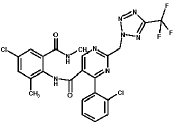 | 3,31 | 565 | (10.313;0.31),(10.306;0.34),(10.279;4.50),(9.170;0.54),(9.134;7.99),(8.332;1.74),(8.321;1.67),(7.522;1.4 8),(7.504;3.64),(7.489;1.82),(7.483;2.00),(7.473;2.16),(7.467;2.72),(7.461;1.62),(7.454;2.16),(7.448;2.7 1),(7.441;5.03),(7.436;5.73),(7.431;6.60),(7.414;2.28),(7.411;2.17),(7.395;0.83),(7.391;0.70),(7.350;4.0 7),(7.344;3.61),(6.604;11.12),(6.394;0.59),(4.039;0.38),(4.021;0.37),(3.303;442.85),(2.691;9.96),(2.680; 9.72),(2.669;1.46),(2.664;1.01),(2.539;2.09),(2.508;49.81),(2.504; 87.03),(2.500;108.65),(2.495;75.66),( 2.335;0.40),(2.331;0.64),(2.326;0.82),(2.322;0.62),(2.069;0.80),(2.040;15.00),(1.987;1.62),(1.398;2.22), (1.237;0.68),(1.224;0.30),(1.192;0.52),(1.175;0.89),(1.157;0.47),(0.890;0.34),(-0.000;7.17),(-0.008;0.37) |

| NMR | Structure | Log p | MH+ | NMR |
|---|---|---|---|---|
| 37 | 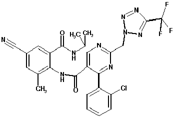 | 3,5 | 584 | (10.506;2.95),(9.149;4.91),(8.326;1.31),(8.307;1.29),(7.826;2.56),(7.762;0.34),(7.739;2.88),(7.735;2.72) ,(7.517;0.93),(7.498;2.65),(7.490;1.56),(7.483;1.42),(7.474;1.46),(7.468;2.16),(7.457;1.24),(7.448;2.44), (7.444;2.81),(7.438;2.84),(7.434;2.11),(7.422;1.28),(7.418;1.24),(7.403;0.46),(7.399;0.40),(6.616;7.82),( 6.407;0.33),(4.039;0.38),(4.021;0.40),(3.960;0.61),(3.943;0.97),(3.925;0.95),(3.909;0.61),(3.301;252.17) ,(2.673;0.51),(2.669;0.67),(2.664;0.51),(2.539;1.64),(2.508;40.24),(2.504;70.87),(2.500;89.15),(2.495;62 .26),(2.331;0.53),(2.326;0.68),(2.322;0.50),(2.286;1.24),(2.126;1.28),(2.089;9.46),(2.069;0.71),(1.987;1. 72),(1.398;8.09),(1.237;0.70),(1.193;0.55),(1.175;1.01),(1.157;0.57),(1.088;15.00),(1.081;4.76),(1.072;1 4.94),(1.065;3.62),(-0.000;5.45) |
| 38 | 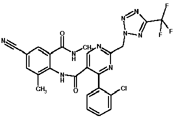 | 2,93 | 556 | (10.544;4.62),(10.173;0.58),(9.188;0.31),(9.149;7.22),(8.418;2.11),(8.407;1.91),(7.997;0.57),(7.900;0.62 ),(7.837;4.14),(7.802;0.63),(7.756;4.51),(7.522;1.71),(7.518;1.63),(7.504;4.47),(7.491;2.20),(7.485;2.14) ,(7.475;2.63),(7.469;3.94),(7.464;2.44),(7.451;5.83),(7.438;2.94),(7.435;2.67),(7.423;2.36),(7.403;0.59), (6.965;0.58),(6.614;11.96),(6.403;0.50),(4.039;0.96),(4.021;0.97),(4.004;0.36),(3.536;0.30),(3.517;0.36) ,(3.502;0.41),(3.483;0.43),(3.311;938.39),(3.288;14.63),(3.230;0.62),(3.206;0.35),(2.693;10.41),(2.682;1 0.28),(2.670;1.90),(2.639;0.33),(2.539;3.24),(2.505;117.82),(2.500;147.75),(2.496;104.00),(2.403;0.34), (2.327;1.07),(2.323;0.77),(2.277;2.49),(2.131;2.72),(2.099;15.00),(2.069;1.63),(2.050;0.44),( 1.987;3.86) ,(1.908;0.78),(1.237;0.55),(1.193;1.05),(1.175;2.08),(1.157;1.11),(0.890;0.32),(-0.000;5.25) |
| 39 | 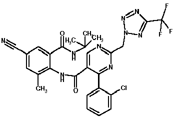 | 3,85 | 598 | (10.470;1.10),(9.121;1.91),(7.999;0.93),(7.795;0.93),(7.717;0.99),(7.713;0.94),(7.525;0.38),(7.506;0.96) ,(7.492;0.38),(7.484;0.41),(7.479;0.41),(7.470;0.61),(7.451;0.65),(7.438;1.64),(7.424;0.51),(6.618;2.84), (3.302;127.15),(3.278;2.95),(2.539;0.66),(2.504;28.83),(2.500;35.92),(2.496;25.60),(2.065;3.49),(1.288; 15.00),(1.276;1.38),(1.263;0.93),(-0.000;2.18) |
| 40 | 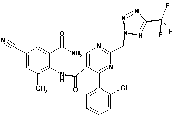 | 2,69 | 542 | (10.590;2.35),(9.193;0.39),(9.183;0.38),(9.156;15.00),(8.041;1.12),(8.005;0.41),(7.988;0.50),(7.926;1.50 ),(7.898;1.33),(7.878;0.85),(7.853;1.74),(7.806;6.03),(7.609;0.36),(7.590;0.48),(7.567;1.01),(7.550;1.09) ,(7.519;4.86),(7.499;6.00),(7.461;7.24),(7.446;7.00),(7.425;2.84),(7.024;0.61),(6.605;12.82),(6.396;0.63 ),(4.057;1.11),(4.039;3.02),(4.021;3.03),(4.004;1.09),(3.457;0.49),(3.430;0.64),(3.424;0.74),(3.305;1129 .01),(3.181;0.50),(3.145;0.42),(2.891;0.38),(2.731;0.39),(2.695;0.40),(2.674; 1.34),(2.669; 1.74),(2.664; 1. 32),(2.622;0.41),(2.615;0.47),(2.594;0.63),(2.579;0.81),(2.539;11.18),(2.508;110.51),(2.504;193.28),(2. 500;241.77),(2.496;168.56),(2.402;0.64),(2.394;0.53),(2.384;0.45),(2.331;1.47),(2.326;1.86),(2.267;2.76 ),(2.245;0.48),(2.214;0.38),(2.199;0.38),(2.188;0.41),(2.175;0.51),(2.170;0.50),(2.130;5.98),(2.109;13.5 9),(2.069;2.56),(2.050;1.25),(2.039;0.62),(2.004;0.47),(1.987;13.11),(1.955;0.45),(1.905;1.06),(1.398;0. 98),(1.299;0.38),(1.293;0.52),(1.260;0.42),(1.23 8;1.01),(1.193;3.67),(1.175;7.14),(1.157;3.56),(0.890;0. 63),(-0.000;5.60) |

| NR | Structure | Log p | MH+ | NMR |
|---|---|---|---|---|
| 41 | 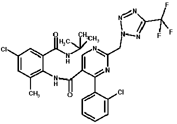 | 4,33 | 607 | (10.253;0.36),(9.108;0.47),(7.980;0.34),(7.513;0.34),(7.433;0.67),(7.401;0.53),(7.286;0.41),(6.623;0.73) ,(3.380;1.74),(3.352;3952.17),(3.328;126.58),(2.621;1.58),(2.618;3.42),(2.615;4.70),(2.612;3.36),(2.609; 1.54),(2.524;9.15),(2.521;11.75),(2.518;11.99),(2.509;248.89),(2.506;532.96),(2.503;720.27),(2.500;512 .66),(2.497;229.26),(2.393;1.41),(2.390;3.23),(2.387;4.47),(2.384;3.12),(2.381;1.32),(2.205;0.31),(2.076 ;13.50),(2.008;1.15),(1.286;3.52),(1.234;0.82),(0.005;1.05),(0.000;31.06),(-0.006;0.87) |
| 42 | 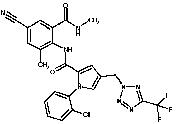 | 3.31 | 543 | (10.13;3.41), (8.42;0.54), (8.41;1.46), (8.40;1.46), (8.40;0.53), (7.82;2.98), (7.82;3.16), (7.73;3.52), (7.73;3.35), (7.54;2.25), (7.53;1.70), (7.53;1.52), (7.52;1.91), (7.52;2.77), (7.44;1.51), (7.44;1.21), (7.44;1.37), (7.43;1.57), (7.43;1.69), (7.43;3.79), (7.42;1.28), (7.41;3.46), (7.41;4.82), (7.40;6.03), (7.40;3.59), (7.39;1.96), (7.38;0.54), (7.35;3.51), (7.35;3.70), (7.24;3.77), (7.23;3.71), (6.05;10.18), (3.57;0.47), (3.36;612.67), (3.34;4.50), (2.66;11.78), (2.65;11.98), (2.62;0.52), (2.62;0.74), (2.61;0.53), (2.54;0.40), (2.52;1.46), (2.52;1.83), (2.52;1.76), (2.51;38.01), (2.51;83.37), (2.50;114.48), (2.50;81.76), (2.50;37.64), (2.39;0.50), (2.39;0.75), (2.38;0.51), (2.17;16.00), (2.08;0.81), (1.99;0.60), (1.17;0.33) |
| 43 | 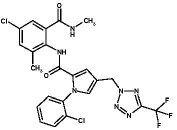 | 3.75 | 552 | (9.78;4.26), (8.21;1.64), (8.20;1.57), (7.53;1.70), (7.52;1.98), (7.52;1.38), (7.51;1.96), (7.51;1.85), (7.51;1.83), (7.50;0.54), (7.43;1.03), (7.42;3.24), (7.41;10.53), (7.40;7.79), (7.39;6.50), (7.38;1.64), (7.37;0.37), (7.31;7.67), (7.31;6.69), (7.19;3.97), (7.18;3.66), (6.04;9.99), (3.49;0.32), (3.48;0.33), (3.46;0.39), (3.44;0.50), (3.31;872.39), (3.29;10.05), (2.83;0.57), (2.74;0.62), (2.67;1.44), (2.65;10.20), (2.64;10.15), (2.58;0.86), (2.54;1.82), (2.51;59.15), (2.50;105.95), (2.50;135.20), (2.50;95.17), (2.49;47.00), (2.43;0.35), (2.33;0.73), (2.33;0.96), (2.32;0.71), (2.16;0.39), (2.13;16.00), (2.11;0.83), (2.07;0.50), (0.00;7.85) |
| 44 | 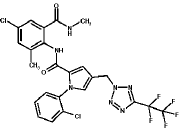 | 4.09 | 602 | (9.81;4.38), (8.23;1.61), (8.22;1.58), (7.53;1.61), (7.53;1.61), (7.52;1.19), (7.52;1.84), (7.51;1.97), (7.50;0.33), (7.44;0.85), (7.42;1.87), (7.42;1.98), (7.41;7.52), (7.41;7.57), (7.41;7.38), (7.41;6.82), (7.40;5.39), (7.39;1.10), (7.38;1.74), (7.32;4.26), (7.32;4.55), (7.31;3.99), (7.31;3.34), (7.19;3.85), (7.18;3.59), (6.07;9.67), (3.32;335.26), (3.30;3.75), (3.08;1.03), (2.67;0.76), (2.67;0.60), (2.65;10.04), (2.64;10.11), (2.54;0.34), (2.52;1.23), (2.51;86.86), (2.50;113.84), (2.50;78.39), (2.33;0.69), (2.13;16.00), (2.07;0.87), (1.24;0.34), (1.11;2.98), (0.00;6.08) |

### Analytische Methoden

Die Bestimmung der in der voranstehenden Tabelle und den Herstellungsbeispielen angegebenen logP Werten erfolgte gemäß EEC Directive 79/831 Annex V.A8 durch HPLC (High Performance Liquid Chromatography) an reversed-phase Säulen (C 18), mit nachfolgenden Methoden:
Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.

Die Kalibrierung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

Die MH⁺-Signale wurden mit einem Agilent MSD-System mit ESI und positiver oder negativer Ionisation bestimmt.

Die NMR-Spektren wurden mit einem Bruker Avance 400, ausgestattet mit einem Durchflussprobenkopf (60 µl Volumen), bestimmt. Als Lösungsmittel wurde d₆-DMSO verwendet, wobei als Referenz Tetramethylsilan (0.00 ppm) eingesetzt wurde. Die Beispiele in obiger Tabelle wurden in d₆-DMSO als Lösungsmittel aufgenommen. Die Meßtemperatur beträgt 303K.

In Einzelfällen wurden die Proben mit einem Bruker Avance II 600 oder III 600 bestimmt.

### Anwendungsbeispiele

### Beispiel 1

**Spodoptera frugiperda-Test (SPODFR Spritzbehandlung)**

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt. Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 83% bei einer Aufwandmenge von 100g/ha: 11

Bei diesem Test zeigen z. B. die folgenden Verbindungen der Herstellungsbeispiele Wirkung von 100% bei einer Aufwandmenge von 100g/ha:
13, 14, 25, 26, 27, 30, 31, 32, 34, 35, 36, 37, 38, 39, 40, 41, 42, 43, 44

### Beispiel 2

**Phaedon-Test (PHAECO Spritzbehandlung)**

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 83% bei einer Aufwandmenge von 100g/ha: 11, 12

Bei diesem Test zeigen z. B. die folgenden Verbindungen der Herstellungsbeispiele Wirkung von 100% bei einer Aufwandmenge von 100g/ha:
10, 13, 25, 26, 27, 30, 32, 34, 35, 36, 37, 38, 39, 40, 41, 42, 43, 44

### Beispiel 3

**Tetranychus-Test; OP-resistent (TETRUR Spritzbehandlung)**

| | | |
|---|---|---|
| Lösungsmittel: | 78,0 | GewichtsteileAceton |
| | 1,5 | Gewichtsteile Dimethylformamid |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 80% bei einer Aufwandmenge von 100g/ha: 8

Bei diesem Test zeigen z. B. die folgenden Verbindungen der Herstellungsbeispiele eine Wirkung von 100 bei einer Aufwandmenge von 100g/ha: 3

### Beispiel 4

**Myzus-Test (MYZUPE Spritzbehandlung)**

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 80% bei einer Aufwandmenge von 100g/ha: 42

Bei diesem Test zeigen z. B. die folgenden Verbindungen der Herstellungsbeispiele Wirkung von 100% bei einer Aufwandmenge von 100g/ha: 43

### Beispiel 5

**Boophilus microplus -Test (BOOPMI Injektion)**

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele eine Wirkung von 100% bei einer Aufwandmenge von 20µg/Tier: 34, 37, 38, 39, 40, 41

Bei diesem Test zeigen z.B. die folgenden Verbindungen der Herstellungsbeispiele eine Wirkung von 98% bei einer Aufwandmenge von 20µg/Tier: 35

### Beispiel 6

**Musca domestica-Test (MUSCDO)**

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die einen Schwamm enthalten, der mit der Wirkstoffzubereitung der gewünschten Konzentration behandelt wurde, werden mit *Musca domestica Adulten* besetzt.

Nach 2 Tagen wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z.B. die folgenden Verbindungen der Herstellungsbeispiele eine Wirkung von 100% bei einer Aufwandmenge von 100ppm: 37

### Beispiel 7

**Lucilia cuprina-Test (LUCICU)**

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die Pferdefleisch enthalten, das mit der Wirkstoffzubereitung der gewünschten Konzentration behandelt wurde, werden mit ca 20 *Lucilia cuprina* Larven besetzt.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: 3, 34, 35, 37, 38, 39, 40, 41

## Patentansprüche

1. Anthranilsäurederivate der Formel (I) 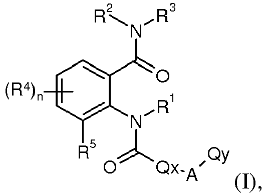 in welcher
in welcher
R¹ für Wasserstoff steht,
R² für Wasserstoff steht.
R³ für Wasserstoff, C₁-C₄-Alkyl (Methyl, Ethyl, n-Propyl, iso-Propyl, n-Butyl, iso-Butyl, sec-Butyl oder tert-Butyl), cyclo-Propyl, cyclo-Butyl, Cyano-C₁-C₃-Alkyl (Cyanomethyl, 1-Cyanoethyl, 2-Cyanoethyl, 1-Cyano-n-Propyl, 2-Cyano-n-Propyl, 3-Cyano-n-Propyl, 1-Cyano-iso-Propyl, 2-Cyano-iso-Propyl), Phenyl, Pyridyl, oder
R⁴ für Wasserstoff, Methyl, Trifluormethyl, Cyano, Fluor, Chlor, Brom, Iod oder Trifluormethoxy steht. Ausserdem stehen zwei benachbarte Reste R⁴ für -(CH₂)₄-, oder -(CH=CH-)₂-.
n für 0 bis 3 steht,
R⁵ für Methyl, Fluor, Chlor, Brom oder Iod steht,
Q_{X} für Thiazol, Oxazol, Pyrrol, Imidazol, Triazol, Pyrimidin, Phenyl oder für Pyrazol steht, wobei der Rest R⁷ über Kohlenstoff an das Pyrazol gebunden ist,
welches durch die Gruppe R⁷ 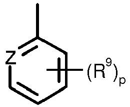 einfach substituiert ist,
A für -CH₂-, -CH(CH₃), C(CH₃)₂, -CH₂CH₂-, -CH(CN)-, -CH₂O- oder -C(=O)-CH₂-steht,
R⁷ für den Rest 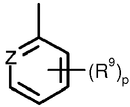
steht,
R⁹ unabhängig voneinander für Fluor, Chlor oder Brom steht,
p für 1 steht,
Z für N, CCl oder CH steht,
Q_{Y} für einen gegebenenfalls einfach oder mehrfach substituierten 5- oder 6-gliedrigen heteroaromatischen Ring der Reihe Q-36 bis Q-40 ,Q43, Q-58 bis Q-59, Q62, Q63, einen aromatisches 9-gliedriges annelliertes heterobicyclisches Ringsystem Q-54 bis Q-56 sowie für ein 5-gliedrigen heterocyclischen Ring Q-60 bis Q-61 steht, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂ C₁-C₄-Alkoxy, C₁-C₄-Haloalkoxy substituiert sein können.

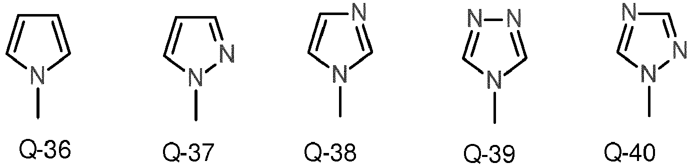

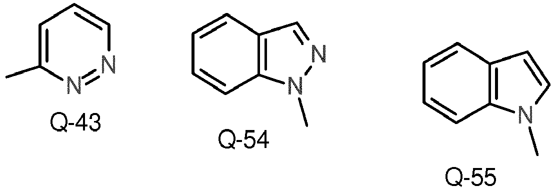

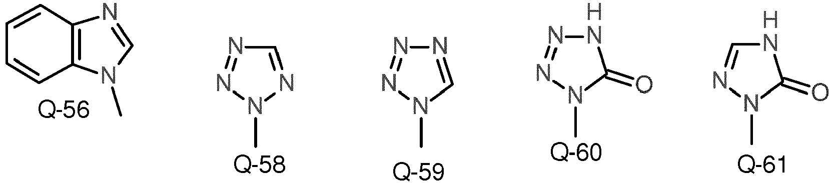

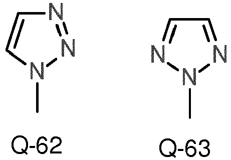

2. Verbindungen der allgemeinen Formel (I) gemäß Anspruch 1, in welcher
R¹ für Wasserstoff steht,
R² für Wasserstoff steht,
R³ für Wasserstoff, Methyl, iso-Propyl, cyclo-Propyl, tert-Butyl oder Cyanomethyl steht,
R⁴ für Chlor, Fluor oder Brom steht,
R⁴ weiterhin für Iod oder Cyano steht; zwei benachbarte Reste R⁴ für -(CH=CH-)₂ stehen,
R⁵ für Methyl, Fluor, Chlor, Brom oder Iod steht,
R⁵ für Methyl oder Chlor steht,
Q_{X} für Thiazol, Oxazol, Pyrrol, Imidazol, Triazol, Pyrimidin, Phenyl oder für Pyrazol steht, wobei der Rest R⁷ über Kohlenstoff an das Pyrazol gebunden ist,
welches durch die Gruppe R⁷ 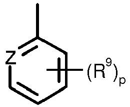 einfach substituiert ist, wobei Z, R und p die oben angegebenen allgemeinen bzw. die unten angegebenen bevorzugten oder besonders bevorzugten Bedeutungen haben können,
A für CH₂, CH(CH₃), -CH₂O- oder -C(=O)-CH₂- steht,
R⁷ für den Rest 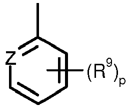
steht,
R⁹ für Chlor steht,
p für 1 steht,
Z für N, CCl oder CH steht,
Q_{Y} für einen gegebenenfalls einfach oder mehrfach gleich oder verschieden substituierten heteroaromatischen Ring der Reihe Q-37, Q-38, Q-39, Q-40, Q43, Q-58, Q-59, Q62 und Q63, sowie für ein 5-gliedrigen heterocyclischen Ring Q-60 steht, wobei die Substituenten unabhängig voneinander ausgewählt sein können aus Methyl, Ethyl, cyclo-Propyl, tert-Butyl, Chlor, Fluor, Iod, Brom, Cyano, Nitro, Difluormethyl, Trifluormethyl, Pentafluorethyl., n-Heptafluorpropyl und iso-Heptafluorpropyl
oder wobei die Substituenten unabhängig voneinander ausgewählt sein können aus Phenyl oder einem 5-oder 6-gliedrigen heteroaromatischen Ring, wobei die Substituenten unabhängig voneinander ausgewählt sein können aus Methyl, Ethyl, cyclo-Propyl, tert-Butyl, Chlor, Fluor, Iod, Brom, Cyano, Nitro, Difluormethyl, Trifluormethyl, Pentafluorethyl., n-Heptafluorpropyl und iso-Heptafluorpropyl.

3. Mischungen von Verbindungen der allgemeinen Formel (I) gemäß einem der Ansprüche 1 bis 2, in welchen Qy für Q62 und Q63 steht, wobei das Verhältnis einer Verbindung der Formel (I), in welchen Qy für Q62 steht, zu einer Verbindung der Formel (I), in welchen Qy für Q63 steht, 60:40 bis 99:1 beträgt.

4. Mischungen von Verbindungen der allgemeinen Formel (I) gemäß einem der Ansprüche 1 bis 2, in welchen Qy für Q58 und Q59 steht, wobei das Verhältnis einer Verbindung der Formel (I), in welchen Qy für Q58 steht, zu einer Verbindung der Formel (I), in welchen Qy für Q59 steht, 60:40 bis 99:1 beträgt.

5. Verfahren zur Herstellung von Verbindungen der allgemeinen Formel (I) gemäß einem der Ansprüche 1 bis 4, **dadurch gekennzeichnet, dass** man
(A) Aniline der Formel (II) 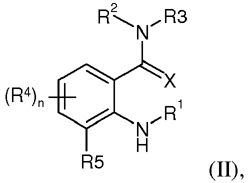 in welcher R¹, R², R³, R⁴, R⁵ und n die oben angegebenen Bedeutungen haben und X für O steht,
beispielsweise mit Carbonsäurechloriden der Formel (III) 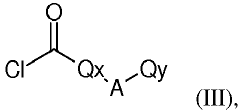 wobei
Qx, A und Qy die oben angegebenen Bedeutungen haben, in Gegenwart eines Säurebindemittels umsetzt; oder
(B) Aniline der Formel (II) 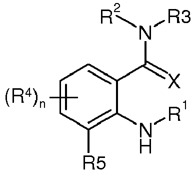 in welcher R¹, R², R³, R⁴, R⁵ und n die oben angegebenen Bedeutungen haben und X für O steht,
beispielsweise mit einer Carbonsäure der Formel (IV) 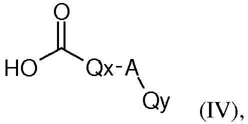 wobei
Qx, A und Qy die oben angegebenen Bedeutungen haben,
in Gegenwart eines Kondensationsmittels umsetzt; oder
(C) zur Synthese von Anthranilamiden der Formel (I), in welcher R¹ für Wasserstoff steht, beispielsweise Benzoxazinone der Formel (V) 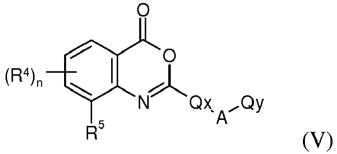 in welcher R⁴, R⁵, Qx, A, Qy und n die oben angegebenen Bedeutungen haben, mit einem Amin der Formel (VI) 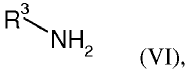 in welcher R³ die oben angegebenen Bedeutungen hat, in Gegenwart eines Verdünnungsmittels umsetzt.

6. Zusammensetzungen enthaltend mindestens eine Verbindung der Formel (I) oder eine Mischung von Verbindungen der Formel (I) gemäß einem der Ansprüche 1 bis 4 sowie mindestens ein Salz der Formel (XXIV) 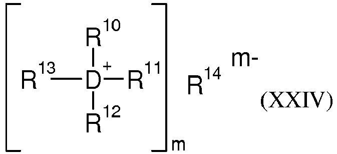 in welcher
D für Stickstoff oder Phosphor steht,
R¹⁰, R¹¹, R¹², and R¹³ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
m für 1, 2, 3 oder 4 steht,
R¹⁴ für ein anorganisches oder organisches Anion steht.

7. Zusammensetzungen enthaltend mindestens eine Verbindung der Formel (I) oder eine Mischung von Verbindungen der Formel (I) gemäß einem der Ansprüche 1 bis 4 sowie mindestens einen Penetrationsförderer der Formel (XXV)
R-O-(-AO)_{V}-R' (XXV),
in welcher
R für geradkettiges oder verzweigtes Alkyl mit 4 bis 20 Kohlenstoffatomen steht,
R' für Wasserstoff, Methyl, Ethyl, n-Propyl, i-Propyl, n-Butyl, i-Butyl, t-Butyl, n-Pentyl oder n-Hexyl steht,
AO für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
V für Zahlen von 2 bis 30 steht.

8. Agrochemische Zusammensetzungen enthaltend mindestens eine Verbindung der Formel (I) oder eine Mischung von Verbindungen der Formel (I) gemäß einem der Ansprüche 1 bis 4 oder eine Zusammensetzung gemäß Anspruch 6 oder 7, sowie Streckmittel und/oder oberflächenaktive Stoffe.

9. Verfahren zur Herstellung agrochemischer Zusammensetzungen, **dadurch gekennzeichnet, dass** mindestens eine Verbindung der allgemeinen Formel (I) oder eine Mischung von Verbindungen der allgemeinen Formel (I) gemäß einem der Ansprüche 1 bis 4 oder eine Zusammensetzung gemäß Anspruch 6 oder 7 mit Streckmitteln und/oder oberflächenaktiven Stoffen gemischt wird.

10. Verwendung einer Verbindung der allgemeinen Formel (I) oder einer Mischung von Verbindungen gemäß einem der Ansprüche 1 bis 4 oder einer Zusammensetzung gemäß einem der Ansprüche 6 bis 8 zur Bekämpfung tierischer Schädlinge, ausgenommen zur therapeutischen Behandlung des tierischen oder menschlichen Körpers.

11. Verfahren zur Bekämpfung tierischer Schädlinge, ausgenommen zur therapeutischen behandlung des tierischen oder menschlichen Körpers, **dadurch gekennzeichnet, dass** man eine Verbindung der allgemeinen Formel (I) oder eine Mischung von Verbindungen der allgemeinen Formel (I) gemäß einem der Ansprüche 1 bis 4 oder eine Zusammensetzung gemäß einem der Ansprüche 6 bis 8 auf tierische Schädlinge und/oder phytopathogene Pilze und/oder deren Lebensraum und/oder Saatgut einwirken lässt

## Claims

1. Anthranilic acid derivatives of the formula (I) 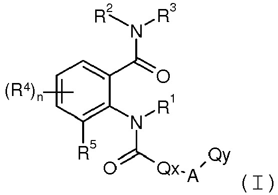 in which
R¹ represents hydrogen,
R² represents hydrogen,
R³ represents hydrogen, C₁-C₄-alkyl (methyl, ethyl, n-propyl, isopropyl, n-butyl, isobutyl, sec-butyl or tert-butyl), cyclopropyl, cyclobutyl, cyano-C₁-C₃-alkyl (cyanomethyl, 1-cyanoethyl, 2-cyanoethyl, 1-cyano-n-propyl, 2-cyano-n-propyl, 3-cyano-n-propyl, 1-cyanoisopropyl, 2-cyanoisopropyl), phenyl, pyridyl or
R⁴ represents hydrogen, methyl, trifluoromethyl, cyano, fluorine, chlorine, bromine, iodine or trifluoromethoxy. Moreover, two adjacent radicals R⁴ represent -(CH₂)₄- or -(CH=CH-)₂-.
n represents 0 to 3,
R⁵ represents methyl, fluorine, chlorine, bromine or iodine,
Qx represents thiazole, oxazole, pyrrole, imidazole, triazole, pyrimidine, phenyl or represents pyrazole, where the radical R⁷ is attached to the pyrazole via carbon,
which is monosubstituted by the group R⁷ 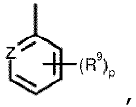
A represents -CH₂-, -CH(CH₃), C(CH₃)₂, -CH₂CH₂-, -CH(CN)-, -CH₂O- or -C(=O)-CH₂,
R⁷ represents the radical 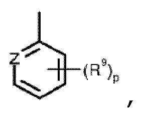
R⁹ independently of one another represents fluorine, chlorine or bromine,
p represents 1,
Z represents N, CC1 or CH,
Q_{Y} represents an optionally mono- or poly-substituted 5- or 6-membered heteroaromatic ring from the group consisting of Q-36 to Q-40, Q43, Q-58 to Q-59, Q-62, Q-63, an aromatic 9-membered fused heterobicyclic ring system Q-54 to Q-56 or a 5-membered hetero-cyclic ring Q-60 to Q-61, where the substituents independently of one another may be selected from C₁-C₃-alkyl, C₁-C₃-haloalkyl, C₁-C₂-alkoxy, halogen, cyano, hydroxy, nitro and C₁-C₂-haloalkoxy,
or where the substituents independently of one another may be selected from the group consisting of phenyl and a 5- or 6-membered heteroaromatic ring, where phenyl or the ring may optionally be mono- or polysubstituted by identical or different substituents from the group consisting of C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, C₁-C₄-alkoxy, C₁-C₄-haloalkoxy.

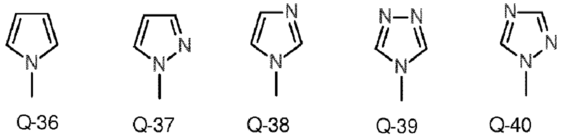

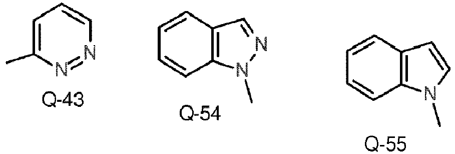

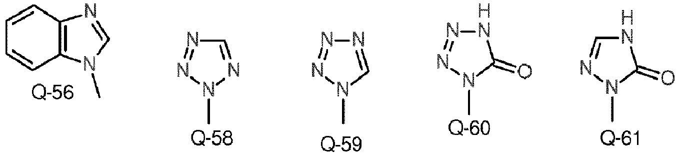

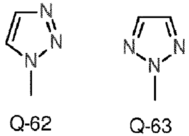

2. Compounds of the general formula (I) according to Claim 1 in which
R¹ represents hydrogen,
R² represents hydrogen,
R³ represents hydrogen, methyl, isopropyl, cyclopropyl, tert-butyl or cyanomethyl,
R⁴ represents chlorine, fluorine or bromine,
R⁴ furthermore represents iodine or cyano; two adjacent radicals R⁴ represent -(CH=CH-)₂,
R⁵ represents methyl, fluorine, chlorine, bromine or iodine,
R⁵ represents methyl or chlorine,
Qx represents thiazole, oxazole, pyrrole, imidazole, triazole, pyrimidine, phenyl or represents pyrazole, where the radical R⁷ is attached to the pyrazole via carbon,
which is monosubstituted by the group R⁷ 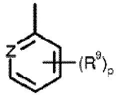 where Z, R and p may have the general meanings given above or the preferred or particularly preferred meanings given below,
A represents CH₂ CH(CH₃), -CH₂O- or -C(=O)-CH₂-,
R⁷ represents the radical 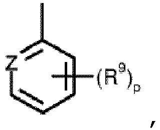
R⁹ represents chlorine,
p represents 1,
Z represents N, CC1 or CH,
Q_{Y} represents an optionally mono- or poly-substituted, by identical or different substituents, heteroaromatic ring from the group consisting of Q-37, Q-38, Q-39, Q-40, Q-43, Q-58, Q-59, Q-62 and Q-63 or represents a 5-membered heterocyclic ring Q-60, where the substituents independently of one another may be selected from methyl, ethyl, cyclopropyl, tert-butyl, chlorine, fluorine, iodine, bromine, cyano, nitro, difluoro-methyl, trifluoromethyl, pentafluoroethyl, n-heptafluoropropyl and isoheptafluoropropyl
or where the substituents independently of one another may be selected from the group consisting of phenyl and a 5- or 6-membered heteroaromatic ring, where the substituents independently of one another may be selected from methyl, ethyl, cyclopropyl, tert-butyl, chlorine, fluorine, iodine, bromine, cyano, nitro, difluoromethyl, trifluoromethyl, pentafluoroethyl, n-heptafluoropropyl and isoheptafluoropropyl.

3. Mixtures of compounds of the general formula (I) according to any of Claims 1 to 2, in which Qy is Q62 and Q63, the ratio of a compound of the formula (I) in which Qy is Q62 to a compound of the formula (I) in which Qy is Q63 being 60:40 to 99:1.

4. Mixtures of compounds of the general formula (I) according to any of Claims 1 to 2, in which Qy is Q58 and Q59, the ratio of a compound of the formula (I) in which Qy is Q58 to a compound of the formula (I) in which Qy is Q59 being 60:40 to 99:1.

5. Process for preparing compounds of the general formula (I) according to any of Claims 1 to 4, **characterized in that**
(A) anilines of the formula (II) 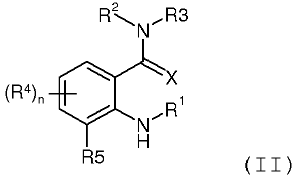 in which R¹, R², R³, R⁴, R⁵ and n have the meanings given above and X represents O,
are reacted, for example, with carbonyl chlorides of the formula (III) 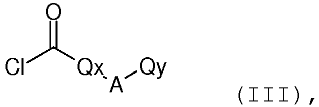 where
Qx, A and Qy have the meanings given above, in the presence of an acid-binding agent; or
(B) anilines of the formula (II) 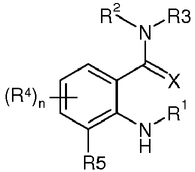 in which R¹, R², R³, R⁴, R⁵ and n have the meanings given above and X represents O,
are reacted, for example, with a carboxylic acid of the formula (IV) 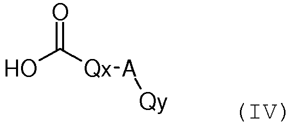 where
Qx, A and Qy have the meanings given above, in the presence of a condensing agent; or
(C) for the synthesis of anthranilamides of the formula (I) in which R¹ represents hydrogen,
for example, benzoxazinones of the formula (V) 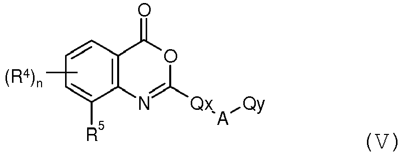 in which R⁴, R⁵, Qx, A, Qy and n have the meanings given above
are reacted with an amine of the formula (VI) 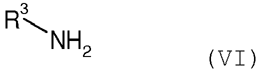 in which R³ has the meanings given above, in the presence of a diluent.

6. Compositions, comprising at least one compound of the formula (I) or a mixture of compounds of the formula (I) according to any of Claims 1 to 4 and at least one salt of the formula (XXIV) 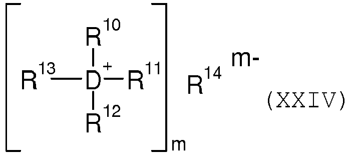 in which
D represents nitrogen or phosphorus,
R¹⁰, R¹¹, R¹² and R¹³ independently of one another represent hydrogen or in each case optionally substituted C₁-C₈-alkyl or mono- or polyunsaturated, optionally substituted C₁-C₈-alkylene where the substituents may be selected from the group consisting of halogen, nitro and cyano,
m represents 1, 2, 3 or 4,
R¹⁴ represents an inorganic or organic anion.

7. Compositions comprising at least one compound of the formula (I) or a mixture of compounds of the formula (I) according to any of Claims 1 to 4 and at least one penetrant of the formula (XXV)
R-O-(-AO)_{V}-R' (XXV)
in which
R represents straight-chain or branched alkyl having 4 to 20 carbon atoms
R' represents hydrogen, methyl, ethyl, n-propyl, isopropyl, n-butyl, isobutyl, tert-butyl, n-pentyl or n-hexyl,
AO represents an ethylene oxide radical, a propylene oxide radical, a butylene oxide radical or represents mixtures of ethylene oxide and propylene oxide radicals or butylene oxide radicals and
V represents a number from 2 to 30.

8. Agrochemical compositions comprising at least one compound of the formula (I) or a mixture of compounds of the formula (I) according to any of Claims 1 to 4 or a composition according to Claim 6 or 7, and also extenders and/or surfactants.

9. Process for producing agrochemical compositions, **characterized in that** at least one compound of the general formula (I) or a mixture of compounds of the general formula (I) according to any of Claims 1 to 4 or a composition according to Claim 6 or 7 is mixed with extenders and/or surfactants.

10. Use of a compound of the general formula (I) or of a mixture of compounds according to any of Claims 1 to 4 or of a composition according to any of Claims 6 to 8 for controlling animal pests, with the exception of for the therapeutic treatment of the animal or human body.

11. Method for controlling animal pests, with the exception of for the therapeutic treatment of the animal or human body, **characterized in that** a compound of the general formula (I) or a mixture of compounds of the general formula (I) according to any of Claims 1 to 4 or a composition according to any of Claims 6 to 8 is applied to animal pests and/or phytopathogenic fungi and/or their habitat and/or seed.

## Revendications

1. Dérivés de l'acide anthranilique de formule (I) 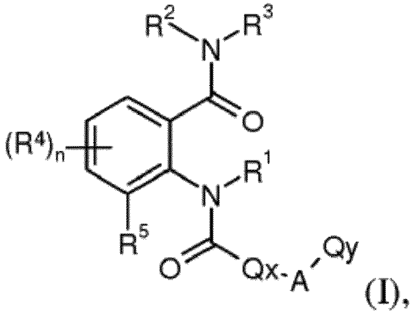 dans laquelle
R¹ représente hydrogène,
R² représente hydrogène,
R³ représente hydrogène, alkyle en C₁-C₄ (méthyle, éthyle, n-propyle, iso-propyle, n-butyle, iso-butyle, sec-butyle ou tert-butyle), cyclo-propyle, cyclo-butyle, cyano-alkyle en C₁-C₃ (cyanométhyle, 1-cyanoéthyle, 2-cyanoéthyle, 1-cyano-n-propyle, 2-cyano-n-propyle, 3-cyano-n-propye, 1-cyano-iso-propyle, 2-cyano-iso-propyle), phényle, pyridyle, ou
R⁴ représente hydrogène, méthyle, trifluorométhyle, cyano, fluor, chlore, brome, iode ou trifluorométhoxy ; deux radicaux R⁴ voisins représentent également -(CH₂)₄-ou -(CH=CH)₂-,
n représente 0 à 3,
R⁵ représente méthyle, fluor, chlore, brome ou iode,
Qₓ représente thiazole, oxazole, pyrrole, imidazole, triazole, pyrimidine, phényle ou pyrazole, le radical R⁷ étant relié par un carbone au pyrazole,
qui est substitué une fois par le groupe R⁷ 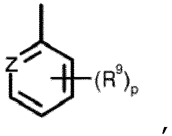
A représente -CH₂-, -CH(CH₃, C(CH₃)₂, -CH₂CH₂-,-CH(CN)-, -CH₂O- ou -C(=O)-CH₂-,
R⁷ représente le radical 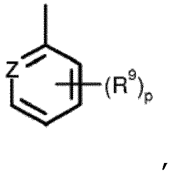
les R⁹ représentent indépendamment les uns des autres fluor, chlore ou brome,
p représente 1,
Z représente N, CCl ou CH,
Q_{y} représente un cycle hétéroaromatique à 5 ou 6 éléments éventuellement mono- ou polysubstitué, de la série Q-36 à Q-40, Q-43, Q-58 à Q-59, Q-62, Q-63, un système cyclique hétérobicyclique annelé aromatique à 9 éléments Q-54 à Q-56, ainsi qu'un cycle hétérocyclique à 5 éléments Q-60 à Q-61, les substituants pouvant être choisis indépendamment les uns des autres parmi alkyle en C₁-C₃, haloalkyle en C₁-C₃, alcoxy en C₁-C₂, halogène, cyano, hydroxy, nitro ou haloalcoxy en C₁-C₂,
ou les substituants pouvant être choisis indépendamment les uns des autres parmi phényle ou un cycle hétéroaromatique à 5 ou 6 éléments, le phényle ou le cycle pouvant éventuellement être substitués une ou plusieurs fois, de manière identique ou différente, avec alkyle en C₁-C₆, alcényle en C₂-C₆, alcynyle en C₂-C₆, cycloalkyle en C₃-C₆, haloalkyle en C₁-C₆, haloalcényle en C₂-C₆, haloalcynyle en C₂-C₆, halocycloalkyle en C₃-C₆, halogène, CN, NO₂, alcoxy en C₁-C₄, haloalcoxy en C₁-C₄,

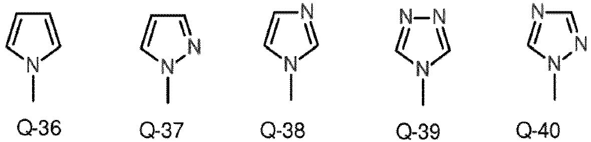

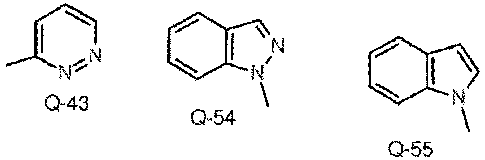

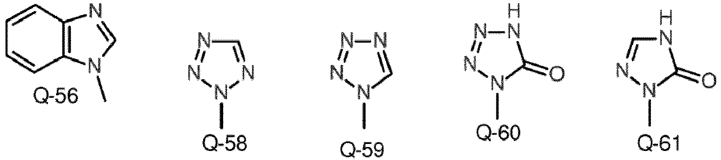

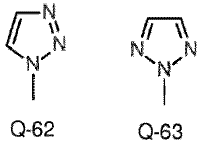

2. Composés de formule générale (I) selon la revendication 1, dans lesquels
R¹ représente hydrogène,
R² représente hydrogène,
R³ représente hydrogène, méthyle, iso-propyle, cyclo-propyle, tert-butyle ou cyanométhyle,
R⁴ représente chlore, fluor ou brome,
R⁴ représente également iode ou cyano ; deux radicaux R⁴ voisins représentent -(CH=CH-)₂,
R⁵ représente méthyle, fluor, chlore, brome ou iode, R⁵ représente méthyle ou chlore,
Qₓ représente thiazole, oxazole, pyrrole, imidazole, triazole, pyrimidine, phényle ou pyrazole, le radical R⁷ étant relié par un carbone au pyrazole,
qui est substitué une fois par le groupe R⁷ 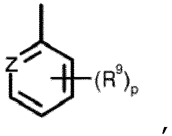 Z, R et p pouvant avoir les significations générales indiquées précédemment ou les significations préférées ou particulièrement préférées indiquées ci-après,
A représente CH₂, CH(CH₃), -CH₂O- ou -C(=O)-CH₂-,
R⁷ représente le radical 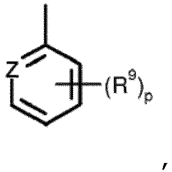
R⁹ représente chlore,
p représente 1,
Z représente N, CCl ou CH,
Q_{y} représente un cycle hétéroaromatique de la série Q-37, Q-38, Q-39, Q-40, Q-43, Q-58, Q-59, Q-62 et Q-63, éventuellement substitué une ou plusieurs fois de manière identique ou différente, ainsi qu'un cycle hétérocyclique à 5 éléments Q-60, les substituants pouvant être choisis indépendamment les uns des autres parmi méthyle, éthyle, cyclo-propyle, tert-butyle, chlore, fluor, iode, brome, cyano, nitro, difluorométhyle, trifluorométhyle, pentafluoroéthyle, n-heptafluoropropyle et iso-heptafluoropropyle,
ou les substituants pouvant être choisis indépendamment les uns des autres parmi phényle ou un cycle hétéroaromatique à 5 ou 6 éléments, les substituants pouvant être choisis indépendamment les uns des autres parmi méthyle, éthyle, cyclo-propyle, tert-butyle, chlore, fluor, iode, brome, cyano, nitro, difluorométhyle, trifluorométhyle, pentafluoroéthyle, n-heptafluoropropyle et iso-heptafluoropropyle.

3. Mélanges de composés de formule générale (I) selon l'une quelconque des revendications 1 à 2, dans lesquels Q_{y} représente Q62 et Q63, le rapport entre un composé de formule (I) dans lequel Q_{y} représente Q62 et un composé de formule (I) dans lequel Q_{y} représente Q63 étant de 60:40 à 99:1.

4. Mélanges de composés de formule générale (I) selon l'une quelconque des revendications 1 à 2, dans lesquels Q_{y} représente Q58 et Q59, le rapport entre un composé de formule (I) dans lequel Q_{y} représente Q58 et un composé de formule (I) dans lequel Q_{y} représente Q59 étant de 60:40 à 99:1.

5. Procédé de fabrication de composés de formule générale (I) selon l'une quelconque des revendications 1 à 4, **caractérisé en ce que**
(A) des anilines de formule (II) 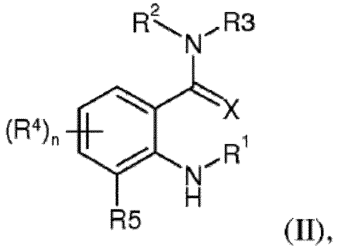 dans laquelle R¹, R², R³, R⁴, R⁵ et n ont les significations indiquées précédemment, et X représente O,
sont mises en réaction par exemple avec des chlorures d'acides carboxyliques de formule (III) 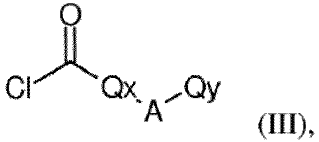 dans laquelle
Qₓ, A et Q_{y} ont les significations indiquées précédemment,
en présence d'un liant d'acide ; ou
(B) des anilines de formule (II) 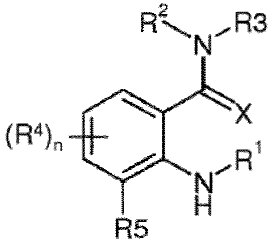 dans laquelle R¹, R², R³, R⁴, R⁵ et n ont les significations indiquées précédemment, et X représente O,
sont mises en réaction par exemple avec un acide carboxylique de formule (IV) 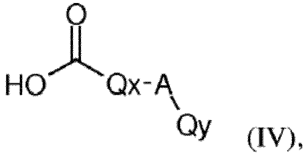 dans laquelle
Qₓ, A et Q_{y} ont les significations indiquées précédemment,
en présence d'un agent de condensation ; ou
(C) pour la synthèse d'anthranilamides de formule (I), dans laquelle R¹ représente l'hydrogène,
par exemple des benzoxazinones de formule (V) 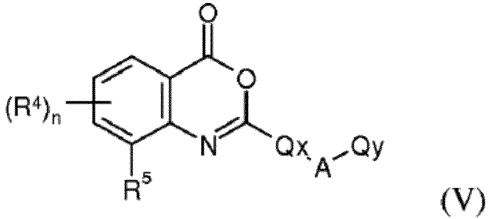 dans laquelle R⁴, R⁵, Qₓ, A, Q_{y} et n ont les significations indiquées précédemment,
sont mises en réaction avec une amine de formule (VI) 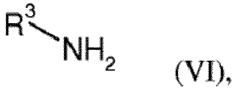 dans laquelle R³ a les significations indiquées précédemment, en présence d'un diluant.

6. Compositions contenant au moins un composé de formule (I) ou un mélange de composés de formule (I) selon l'une quelconque des revendications 1 à 4, ainsi qu'au moins un sel de formule (XXIV) 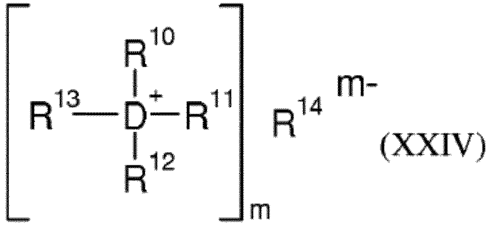 dans laquelle
D représente azote ou phosphore,
R¹⁰, R¹¹, R¹² et R¹³ représentent indépendamment les uns des autres hydrogène ou alkyle en C₁-C₈ à chaque fois éventuellement substitué, ou alkylène en C₁-C₈ mono- ou polyinsaturé, éventuellement substitué, les substituants pouvant être choisis parmi halogène, nitro et cyano,
m représente 1, 2, 3 ou 4,
R¹⁴ représente un anion inorganique ou organique.

7. Compositions contenant au moins un composé de formule (I) ou un mélange de composés de formule (I) selon l'une quelconque des revendications 1 à 4, ainsi qu'au moins un promoteur de pénétration de formule (XXV)
R-O-(-AO)_{V}-R' (XXV)
dans laquelle
R représente alkyle linéaire ou ramifié de 4 à 20 atomes de carbone,
R' représente hydrogène, méthyle, éthyle, n-propyle, i-propyle, n-butyle, i-butyle, t-butyle, n-pentyle ou n-hexyle,
AO représente un radical oxyde d'éthylène, un radical oxyde de propylène, un radical oxyde de butylène ou des mélanges de radicaux oxyde d'éthylène et oxyde de propylène ou de radicaux oxyde de butylène, et
V représente des nombres de 2 à 30.

8. Compositions agrochimiques contenant au moins un composé de formule (I) ou un mélange de composés de formule (I) selon l'une quelconque des revendications 1 à 4 ou une composition selon la revendication 6 ou 7, ainsi que des extendeurs et/ou des substances tensioactives.

9. Procédé de fabrication de compositions agrochimiques, **caractérisé en ce qu'**au moins un composé de formule générale (I) ou un mélange de composés de formule générale (I) selon l'une quelconque des revendications 1 à 4 ou une composition selon la revendication 6 ou 7 est mélangé avec des extendeurs et/ou des substances tensioactives.

10. Utilisation d'un composé de formule générale (I) ou d'un mélange de composés selon l'une quelconque des revendications 1 à 4 ou d'une composition selon l'une quelconque des revendications 6 à 8 pour lutter contre des animaux nuisibles, à l'exception du traitement thérapeutique du corps animal ou humain.

11. Procédé de lutte contre des animaux nuisibles, à l'exception du traitement thérapeutique du corps animal ou humain, **caractérisé en ce qu'**un composé de formule générale (I) ou un mélange de composés de formule générale (I) selon l'une quelconque des revendications 1 à 4 ou une composition selon l'une quelconque des revendications 6 à 8 est laissé agir sur des animaux nuisibles et/ou des champignons phytopathogènes et/ou leur habitat et/ou des graines.
